# EUROPEAN PATENT APPLICATION

(11) **EP 1 782 822 A1**
(43) Date of publication of application: **09.05.2007**
(21) Application number: 05768479.7
(22) Date of filing: 03.08.2005
(51) Int. Cl.: A61K 38/00, A61P 35/00, C12Q 1/02, C12N 15/00

(54) **REMEDY FOR MELAONOMA**

(30) Priority: 04.08.2004 JP 2004228294
(71) Applicant: DAIICHI PHARMACEUTICAL CO., LTD., Chuo-ku, Tokyo 103-8234 (JP)
(72) Inventor: DOI, Hirofumi, c/o Celestar Lexico-Sciences, Inc., Chiba-shi, Chiba 261-8501 (JP); SAITO, Seiji, c/o Celestar Lexico-Sciences, Inc., Chiba-shi, Chiba 261-8501 (JP); MURAKAMI, Kenji., Daiichi Pharmaceutical Co., Ltd., Ed ogawa-ku, Tokyo 134-8630 (JP); KURITA, Yukako, 1320014 (JP)
(74) Representative: De Clercq, Ann G. Y.
(86) International application number: PCT/JP2005/014199
(87) International publication number: WO 2006/013889

(57) **Abstract**

The present invention was aimed at reducing transcription activating activity of MTTF-M to inhibit production of BCL2 gene product and thereby to allow treatment and/or prevention of melanoma. The present invention provided a method of inhibiting production of BCL2 gene product and an agent for inhibiting the same, which inhibit binding ofprotein selected from a group consisting of HLF, ELK4 and CLOCK to MITF-M, a method of inducing cell death of melanoma cells, an agent for inducing the same, an agent for treating and/or preventing diseases accompanied by enhanced production of BCL2 gene product, such as melanoma, a method of treating and/or preventing the diseases, a method of identifying any one of the following compounds: a compound that inhibits the aforementioned binding; a compound that inhibits production of BCL2 gene product; and a compound that increases sensitivity of melanoma to melanoma drugs, as well as a reagent kit.

SEQUENCE LISTING

<110>  DAIICHI PHARMACEUTICAL CO., LTD.

<120>  Remedy for melanoma

<130>  DAII-006-EP

<140>  05768479.7
<141>  2005-08-03

<150>  JP 2004-228294
<151>  2004-08-04

<160>  38

<170>  PatentIn version 3.3

<210>  1
<211>  1260
<212>  DNA
<213>  Homo sapiens

<220>
<221>  misc_feature
<223>  A gene encoding MITF-M (SEQ ID NO:2).

<220>
<221>  CDS
<222>  (1)..(1260)

<400>  1
atg ctg gaa atg cta gaa tat aat cac tat cag gtg cag acc cac ctc      48
gaa aac ccc acc aag tac cac ata cag caa gcc caa cgg cag cag gta      96
aag cag tac ctt tct acc act tta gca aat aaa cat gcc aac caa gtc      144
ctg agc ttg cca tgt cca aac cag cct ggc gat cat gtc atg cca ccg      192
gtg ccg ggg agc agc gca ccc aac agc ccc atg gct atg ctt acg ctt      240
aac tcc aac tgt gaa aaa gag gga ttt tat aag ttt gaa gag caa aac      288
agg gca gag agc gag tgc cca ggc atg aac aca cat tca cga gcg tcc      336
tgt atg cag atg gat gat gta atc gat gac atc att agc cta gaa tca      384
agt tat aat gag gaa atc ttg ggc ttg atg gat cct gct ttg caa atg      432
gca aat acg ttg cct gtc tcg gga aac ttg att gat ctt tat gga aac      480
caa ggt ctg ccc cca cca ggc ctc acc atc agc aac tcc tgt cca gcc      528
aac ctt ccc aac ata aaa agg gag ctc aca gcg tgt att ttt ccc aca 576
gag tct gaa gca aga gca ctg gcc aaa gag agg cag aaa aag gac aat 624
cac aac ctg att gaa cga aga aga aga ttt aac ata aat gac cgc att 672
aaa gaa cta ggt act ttg att ccc aag tca aat gat cca gac atg cgc 720
tgg aac aag gga acc atc tta aaa gca tcc gtg gac tat atc cga aag 768
ttg caa cga gaa cag caa cgc gca aaa gaa ctt gaa aac cga cag aag 816
aaa ctg gag cac gcc aac cgg cat ttg ttg ctc aga ata cag gaa ctt 864
gaa atg cag gct cga gct cat gga ctt tcc ctt att cca tcc acg ggt 912
ctc tgc tct cca gat ttg gtg aat cgg atc atc aag caa gaa ccc gtt 960
ctt gag aac tgc agc caa gac ctc ctt cag cat cat gca gac cta acc 1008
tgt aca aca act ctc gat ctc acg gat ggc acc atc acc ttc aac aac 1056
aac ctc gga act ggg act gag gcc aac caa gcc tat agt gtc ccc aca 1104
aaa atg gga tcc aaa ctg gaa gac atc ctg atg gac gac acc ctt tct 1152
ccc gtc ggt gtc act gat cca ctc ctt tcc tca gtg tcc ccc gga gct 1200
tcc aaa aca agc agc cgg agg agc agt atg agc atg gaa gag acg gag 1248
cac act tgt tag 1260

<210> 2
<211> 419
<212> PRT
<213> Homo sapiens

<400> 2

<210>  3
<211>  888
<212>  DNA
<213>  Homo sapiens

<220>
<221>  misc_feature
<223>  A gene encoding HLF (SEQ ID NO:4).

<220>
<221>  CDS
<222>  (1)..(888)

<400>  3
atg gag aaa atg tcc cga ccg ctc ccc ctg aat ccc acc ttt atc ccg      48
Met Glu Lys Met Ser Arg Pro Leu Pro Leu Asn Pro Thr Phe Ile Pro
1               5                   10                  15

cct ccc tac ggc gtg ctc agg tcc ctg ctg gag aac ccg ctg aag ctc      96
Pro Pro Tyr Gly Val Leu Arg Ser Leu Leu Glu Asn Pro Leu Lys Leu
            20                  25                  30

ccc ctt cac cac gaa gac gca ttt agt aaa gat aaa gac aag gaa aag     144
Pro Leu His His Glu Asp Ala Phe Ser Lys Asp Lys Asp Lys Glu Lys
        35                  40                  45

aag ctg gat gat gag agt aac agc ccg acg gtc ccc cag tcg gca ttc     192
Lys Leu Asp Asp Glu Ser Asn Ser Pro Thr Val Pro Gln Ser Ala Phe
    50                  55                  60

ctg ggg cct acc tta tgg gac aaa acc ctt ccc tat gac gga gat act     240
Leu Gly Pro Thr Leu Trp Asp Lys Thr Leu Pro Tyr Asp Gly Asp Thr
65                  70                  75                  80

ttc cag ttg gaa tac atg gac ctg gag gag ttt ttg tca gaa aat ggc     288
Phe Gln Leu Glu Tyr Met Asp Leu Glu Glu Phe Leu Ser Glu Asn Gly
                85                  90                  95

att ccc ccc agc cca tct cag cat gac cac agc cct cac cct cct ggg     336
Ile Pro Pro Ser Pro Ser Gln His Asp His Ser Pro His Pro Pro Gly
            100                 105                 110

ctg cag cca gct tcc tcg gct gcc ccc tcg gtc atg gac ctc agc agc     384
Leu Gln Pro Ala Ser Ser Ala Ala Pro Ser Val Met Asp Leu Ser Ser
        115                 120                 125

cgg gcc tct gca ccc ctt cac cct ggc atc cca tct ccg aac tgt atg 432
Arg Ala Ser Ala Pro Leu His Pro Gly Ile Pro Ser Pro Asn Cys Met
    130                 135                 140

cag agc ccc atc aga cca ggt cag ctg ttg cca gca aac cgc aat aca 480
Gln Ser Pro Ile Arg Pro Gly Gln Leu Leu Pro Ala Asn Arg Asn Thr
145                 150                 155                 160

cca agt ccc att gat cct gac acc atc cag gtc cca gtg ggt tat gag 528
Pro Ser Pro Ile Asp Pro Asp Thr Ile Gln Val Pro Val Gly Tyr Glu
                165                 170                 175

cca gac cca gca gat ctt gcc ctt tcc agc atc cct ggc cag gaa atg 576
Pro Asp Pro Ala Asp Leu Ala Leu Ser Ser Ile Pro Gly Gln Glu Met
            180                 185                 190

ttt gac cct cgc aaa cgc aag ttc tct gag gaa gaa ctg aag cca cag 624
Phe Asp Pro Arg Lys Arg Lys Phe Ser Glu Glu Glu Leu Lys Pro Gln
        195                 200                 205

ccc atg atc aag aaa gct cgc aaa gtc ttc atc cct gat gac ctg aag 672
Pro Met Ile Lys Lys Ala Arg Lys Val Phe Ile Pro Asp Asp Leu Lys
    210                 215                 220

gat gac aag tac tgg gca agg cgc aga aag aac aac atg gca gcc aag 720
Asp Asp Lys Tyr Trp Ala Arg Arg Arg Lys Asn Asn Met Ala Ala Lys
225                 230                 235                 240

cgc tcc cgc gac gcc cgg agg ctg aaa gag aac cag atc gcc atc cgg 768
Arg Ser Arg Asp Ala Arg Arg Leu Lys Glu Asn Gln Ile Ala Ile Arg
                245                 250                 255

gcc tcg ttc ctg gag aag gag aac tcg gcc ctc cgc cag gag gtg gct 816
Ala Ser Phe Leu Glu Lys Glu Asn Ser Ala Leu Arg Gln Glu Val Ala
            260                 265                 270

gac ttg agg aag gag ctg ggc aaa tgc aag aac ata ctt gcc aag tat 864
Asp Leu Arg Lys Glu Leu Gly Lys Cys Lys Asn Ile Leu Ala Lys Tyr
        275                 280                 285

gag gcc agg cac ggg ccc ctg tag 888
Glu Ala Arg His Gly Pro Leu
    290                 295

<210> 4
<211> 295
<212> PRT
<213> Homo sapiens

<400> 4
   Met Glu Lys Met Ser Arg Pro Leu Pro Leu Asn Pro Thr Phe Ile Pro
   1 5 10 15
   Pro Pro Tyr Gly Val Leu Arg Ser Leu Leu Glu Asn Pro Leu Lys Leu
   20 25 30
   Pro Leu His His Glu Asp Ala Phe Ser Lys Asp Lys Asp Lys Glu Lys
   35 40 45
   Lys Leu Asp Asp Glu Ser Asn Ser Pro Thr Val Pro Gln Ser Ala Phe
   50 55 60
   Leu Gly Pro Thr Leu Trp Asp Lys Thr Leu Pro Tyr Asp Gly Asp Thr
   65 70 75 80
   Phe Gln Leu Glu Tyr Met Asp Leu Glu Glu Phe Leu Ser Glu Asn Gly
   85 90 95
   Ile Pro Pro Ser Pro Ser Gln His Asp His Ser Pro His Pro Pro Gly
               100                 105                 110
   Leu Gln Pro Ala Ser Ser Ala Ala Pro Ser Val Met Asp Leu Ser Ser
           115                 120                 125
   Arg Ala Ser Ala Pro Leu His Pro Gly Ile Pro Ser Pro Asn Cys Met
       130                 135                 140
   Gln Ser Pro Ile Arg Pro Gly Gln Leu Leu Pro Ala Asn Arg Asn Thr
   145                 150                 155                 160
   Pro Ser Pro Ile Asp Pro Asp Thr Ile Gln Val Pro Val Gly Tyr Glu
                   165                 170                 175
   Pro Asp Pro Ala Asp Leu Ala Leu Ser Ser Ile Pro Gly Gln Glu Met
               180                 185                 190
   Phe Asp Pro Arg Lys Arg Lys Phe Ser Glu Glu Glu Leu Lys Pro Gln
           195                 200                 205
   Pro Met Ile Lys Lys Ala Arg Lys Val Phe Ile Pro Asp Asp Leu Lys
       210                 215                 220
   Asp Asp Lys Tyr Trp Ala Arg Arg Arg Lys Asn Asn Met Ala Ala Lys
   225                 230                 235                 240
   Arg Ser Arg Asp Ala Arg Arg Leu Lys Glu Asn Gln Ile Ala Ile Arg
                   245                 250                 255
   Ala Ser Phe Leu Glu Lys Glu Asn Ser Ala Leu Arg Gln Glu Val Ala
               260                 265                 270
   Asp Leu Arg Lys Glu Leu Gly Lys Cys Lys Asn Ile Leu Ala Lys Tyr
           275                 280                 285
   Glu Ala Arg His Gly Pro Leu
       290                 295

<210> 5
<211> 1218
<212> DNA
<213> Homo sapiens

<220>
<221> misc_feature
<223> A gene encoding ELK4 (SEQ ID NO:6).

<220>
<221> CDS
<222> (1)..(1218)

<400> 5
atg gac agt gct atc acc ctg tgg cag ttc ctt ctt cag ctc ctg cag 48
Met Asp Ser Ala Ile Thr Leu Trp Gln Phe Leu Leu Gln Leu Leu Gln
1               5                   10                  15

aag cct cag aac aag cac atg atc tgt tgg acc tct aat gat ggg cag 96
Lys Pro Gln Asn Lys His Met Ile Cys Trp Thr Ser Asn Asp Gly Gln
            20                  25                  30

ttt aag ctt ttg cag gca gaa gag gtg gct cgt ctc tgg ggg att cgc 144

Phe Lys Leu Leu Gln Ala Glu Glu Val Ala Arg Leu Trp Gly Ile Arg
        35                  40                  45

aag aac aag cct aac atg aat tat gac aaa ctc agc cga gcc ctc aga        192
Lys Asn Lys Pro Asn Met Asn Tyr Asp Lys Leu Ser Arg Ala Leu Arg
    50                  55                  60

tac tat tat gta aag aat atc atc aaa aaa gtg aat ggt cag aag ttt        240
Tyr Tyr Tyr Val Lys Asn Ile Ile Lys Lys Val Asn Gly Gln Lys Phe
65                  70                  75                  80

gtg tac aag ttt gtc tct tat cca gag att ttg aac atg gat cca atg        288
Val Tyr Lys Phe Val Ser Tyr Pro Glu Ile Leu Asn Met Asp Pro Met
                85                  90                  95

aca gtg ggc agg att gag ggt gac tgt gaa agt tta aac ttc agt gaa        336
Thr Val Gly Arg Ile Glu Gly Asp Cys Glu Ser Leu Asn Phe Ser Glu
            100                 105                 110

gtc agc agc agt tcc aaa gat gtg gag aat gga ggg aaa gat aaa cca        384
Val Ser Ser Ser Ser Lys Asp Val Glu Asn Gly Gly Lys Asp Lys Pro
        115                 120                 125

cct cag cct ggt gcc aag acc tct agc cgc aat gac tac ata cac tct        432
Pro Gln Pro Gly Ala Lys Thr Ser Ser Arg Asn Asp Tyr Ile His Ser
    130                 135                 140

ggc tta tat tct tca ttt act ctc aac tct ttg aac tcc tcc aat gta        480
Gly Leu Tyr Ser Ser Phe Thr Leu Asn Ser Leu Asn Ser Ser Asn Val
145                 150                 155                 160

aag ctt ttc aaa ttg ata aag act gag aat cca gcc gag aaa ctg gca        528
Lys Leu Phe Lys Leu Ile Lys Thr Glu Asn Pro Ala Glu Lys Leu Ala
                165                 170                 175

gag aaa aaa tct cct cag gag ccc aca cca tct gtc atc aaa ttt gtc        576
Glu Lys Lys Ser Pro Gln Glu Pro Thr Pro Ser Val Ile Lys Phe Val
            180                 185                 190

acg aca cct tcc aaa aag cca ccg gtt gaa cct gtt gct gcc acc att        624
Thr Thr Pro Ser Lys Lys Pro Pro Val Glu Pro Val Ala Ala Thr Ile
        195                 200                 205

tca att ggc cca agt att tct cca tct tca gaa gaa act atc caa gct        672
Ser Ile Gly Pro Ser Ile Ser Pro Ser Ser Glu Glu Thr Ile Gln Ala
    210                 215                 220

ttg gag aca ttg gtt tcc cca aaa ctg cct tcc ctg gaa gcc cca acc        720
Leu Glu Thr Leu Val Ser Pro Lys Leu Pro Ser Leu Glu Ala Pro Thr
225                 230                 235                 240

tct gcc tct aac gta atg act gct ttt gcc acc aca cca ccc att tcg        768
Ser Ala Ser Asn Val Met Thr Ala Phe Ala Thr Thr Pro Pro Ile Ser
                245                 250                 255

tcc ata ccc cct ttg cag gaa cct ccc aga aca cct tca cca cca ctg        816
Ser Ile Pro Pro Leu Gln Glu Pro Pro Arg Thr Pro Ser Pro Pro Leu
            260                 265                 270

agt tct cac cca gac atc gac aca gac att gat tca gtg gct tct cag        864
Ser Ser His Pro Asp Ile Asp Thr Asp Ile Asp Ser Val Ala Ser Gln
        275                 280                 285

cca atg gaa ctt cca gag aat ttg tca ctg gag cct aaa gac cag gat        912
Pro Met Glu Leu Pro Glu Asn Leu Ser Leu Glu Pro Lys Asp Gln Asp
    290                 295                 300

tca gtc ttg cta gaa aag gac aaa gta aat aat tca tca aga tcc aag        960
Ser Val Leu Leu Glu Lys Asp Lys Val Asn Asn Ser Ser Arg Ser Lys
305                 310                 315                 320

aaa ccc aaa ggg tta gaa ctg gca ccc acc ctt gtg atc acg agc agt       1008
Lys Pro Lys Gly Leu Glu Leu Ala Pro Thr Leu Val Ile Thr Ser Ser
                325                 330                 335

gat cca agc cca ctg gga ata ctg agc cca tct ctc cct aca gct tct       1056

Asp Pro Ser Pro Leu Gly Ile Leu Ser Pro Ser Leu Pro Thr Ala Ser
            340                 345                 350

ctt aca cca gca ttt ttt tca cag gta gct tgc tcg ctc ttt atg gtg      1104
Leu Thr Pro Ala Phe Phe Ser Gln Val Ala Cys Ser Leu Phe Met Val
        355                 360                 365

tca cca ttg ctt tca ttt att tgc cct ttt aag caa atc cag aat tta      1152
Ser Pro Leu Leu Ser Phe Ile Cys Pro Phe Lys Gln Ile Gln Asn Leu
    370                 375                 380

tac act caa gtt tgc ttt ctg tta ctt agg ttt gtc tta gaa agg tta      1200
Tyr Thr Gln Val Cys Phe Leu Leu Leu Arg Phe Val Leu Glu Arg Leu
385                 390                 395                 400

tgt gtg act gtc atg tga                                              1218
Cys Val Thr Val Met
                405


<210>  6
<211>  405
<212>  PRT
<213>  Homo sapiens

<400>  6

Met Asp Ser Ala Ile Thr Leu Trp Gln Phe Leu Leu Gln Leu Leu Gln
1               5                   10                  15

Lys Pro Gln Asn Lys His Met Ile Cys Trp Thr Ser Asn Asp Gly Gln
            20                  25                  30

Phe Lys Leu Leu Gln Ala Glu Glu Val Ala Arg Leu Trp Gly Ile Arg
        35                  40                  45

Lys Asn Lys Pro Asn Met Asn Tyr Asp Lys Leu Ser Arg Ala Leu Arg
    50                  55                  60

Tyr Tyr Tyr Val Lys Asn Ile Ile Lys Lys Val Asn Gly Gln Lys Phe
65                  70                  75                  80

Val Tyr Lys Phe Val Ser Tyr Pro Glu Ile Leu Asn Met Asp Pro Met
                85                  90                  95

Thr Val Gly Arg Ile Glu Gly Asp Cys Glu Ser Leu Asn Phe Ser Glu
            100                 105                 110

Val Ser Ser Ser Ser Lys Asp Val Glu Asn Gly Gly Lys Asp Lys Pro
        115                 120                 125

Pro Gln Pro Gly Ala Lys Thr Ser Ser Arg Asn Asp Tyr Ile His Ser
    130                 135                 140

Gly Leu Tyr Ser Ser Phe Thr Leu Asn Ser Leu Asn Ser Ser Asn Val
145                 150                 155                 160

Lys Leu Phe Lys Leu Ile Lys Thr Glu Asn Pro Ala Glu Lys Leu Ala
                165                 170                 175

Glu Lys Lys Ser Pro Gln Glu Pro Thr Pro Ser Val Ile Lys Phe Val
            180                 185                 190

Thr Thr Pro Ser Lys Lys Pro Pro Val Glu Pro Val Ala Ala Thr Ile

195                 200                 205

Ser Ile Gly Pro Ser Ile Ser Pro Ser Ser Glu Glu Thr Ile Gln Ala
    210                 215                 220

Leu Glu Thr Leu Val Ser Pro Lys Leu Pro Ser Leu Glu Ala Pro Thr
225                 230                 235                 240

Ser Ala Ser Asn Val Met Thr Ala Phe Ala Thr Thr Pro Pro Ile Ser
                245                 250                 255

Ser Ile Pro Pro Leu Gln Glu Pro Pro Arg Thr Pro Ser Pro Pro Leu
            260                 265                 270

Ser Ser His Pro Asp Ile Asp Thr Asp Ile Asp Ser Val Ala Ser Gln
        275                 280                 285

Pro Met Glu Leu Pro Glu Asn Leu Ser Leu Glu Pro Lys Asp Gln Asp
    290                 295                 300

Ser Val Leu Leu Glu Lys Asp Lys Val Asn Asn Ser Ser Arg Ser Lys
305                 310                 315                 320

Lys Pro Lys Gly Leu Glu Leu Ala Pro Thr Leu Val Ile Thr Ser Ser
                325                 330                 335

Asp Pro Ser Pro Leu Gly Ile Leu Ser Pro Ser Leu Pro Thr Ala Ser
            340                 345                 350

Leu Thr Pro Ala Phe Phe Ser Gln Val Ala Cys Ser Leu Phe Met Val
        355                 360                 365

Ser Pro Leu Leu Ser Phe Ile Cys Pro Phe Lys Gln Ile Gln Asn Leu
    370                 375                 380

Tyr Thr Gln Val Cys Phe Leu Leu Leu Arg Phe Val Leu Glu Arg Leu
385                 390                 395                 400

Cys Val Thr Val Met
                405

<210>  7
<211>  2541
<212>  DNA
<213>  Homo sapiens

<220>
<221>  misc_feature
<223>  A gene encoding CLOCK (SEQ ID NO:8).

<220>
<221>  CDS
<222>  (1)..(2541)

<400>  7
atg ttg ttt acc gta agc tgt agt aaa atg agc tcg att gtt gac aga       48
Met Leu Phe Thr Val Ser Cys Ser Lys Met Ser Ser Ile Val Asp Arg
1               5                   10                  15

gat gac agt agt att ttt gat ggg ttg gtg gaa gaa gat gac aag gac       96
Asp Asp Ser Ser Ile Phe Asp Gly Leu Val Glu Glu Asp Asp Lys Asp

                20                  25                  30

aaa gcg aaa aga gta tct aga aac aaa tct gaa aag aaa cgt aga gat          144
Lys Ala Lys Arg Val Ser Arg Asn Lys Ser Glu Lys Lys Arg Arg Asp
    35                  40                  45

caa ttt aat gtt ctc att aaa gaa ctg gga tcc atg ctt cct ggt aat          192
Gln Phe Asn Val Leu Ile Lys Glu Leu Gly Ser Met Leu Pro Gly Asn
50                  55                  60

gct aga aag atg gac aaa tct act gtt ctg cag aaa agc att gat ttt          240
Ala Arg Lys Met Asp Lys Ser Thr Val Leu Gln Lys Ser Ile Asp Phe
65                  70                  75                  80

tta cga aaa cat aaa gaa atc act gca cag tca gat gct agt gaa att          288
Leu Arg Lys His Lys Glu Ile Thr Ala Gln Ser Asp Ala Ser Glu Ile
                85                  90                  95

cga cag gac tgg aaa cct aca ttc ctt agt aat gaa gag ttt aca caa          336
Arg Gln Asp Trp Lys Pro Thr Phe Leu Ser Asn Glu Glu Phe Thr Gln
            100                 105                 110

tta atg tta gag gct ctt gat ggt ttt ttt tta gca atc atg aca gat          384
Leu Met Leu Glu Ala Leu Asp Gly Phe Phe Leu Ala Ile Met Thr Asp
        115                 120                 125

gga agc ata ata tat gtg tct gag agt gta act tca tta ctt gaa cat          432
Gly Ser Ile Ile Tyr Val Ser Glu Ser Val Thr Ser Leu Leu Glu His
    130                 135                 140

tta cca tct gat ctt gtg gat caa agt ata ttt aat ttt atc cca gaa          480
Leu Pro Ser Asp Leu Val Asp Gln Ser Ile Phe Asn Phe Ile Pro Glu
145                 150                 155                 160

ggg gaa cat tca gag gtt tat aaa ata ctc tct act cat ctg ctg gaa          528
Gly Glu His Ser Glu Val Tyr Lys Ile Leu Ser Thr His Leu Leu Glu
                165                 170                 175

agt gat tca tta acc cca gaa tat tta aaa tca aaa aat cag tta gaa          576
Ser Asp Ser Leu Thr Pro Glu Tyr Leu Lys Ser Lys Asn Gln Leu Glu
            180                 185                 190

ttc tgt tgt cac atg ctg cga gga aca ata gac cca aag gag cca tct          624
Phe Cys Cys His Met Leu Arg Gly Thr Ile Asp Pro Lys Glu Pro Ser
        195                 200                 205

acc tat gaa tat gta aaa ttt ata gga aat ttc aaa tct tta aac agt          672
Thr Tyr Glu Tyr Val Lys Phe Ile Gly Asn Phe Lys Ser Leu Asn Ser
    210                 215                 220

gta tcc tct tca gca cac aat ggt ttt gaa gga act ata caa cgc aca          720
Val Ser Ser Ser Ala His Asn Gly Phe Glu Gly Thr Ile Gln Arg Thr
225                 230                 235                 240

cat agg cca tct tat gaa gat aga gtt tgt ttt gta gct act gtc agg          768
His Arg Pro Ser Tyr Glu Asp Arg Val Cys Phe Val Ala Thr Val Arg
                245                 250                 255

tta gct aca cct cag ttc atc aag gaa atg tgc act gtt gaa gaa ccc          816
Leu Ala Thr Pro Gln Phe Ile Lys Glu Met Cys Thr Val Glu Glu Pro
            260                 265                 270

aat gaa gag ttt aca tct aga cat agt tta gaa tgg aag ttt ctg ttt          864
Asn Glu Glu Phe Thr Ser Arg His Ser Leu Glu Trp Lys Phe Leu Phe
        275                 280                 285

cta gat cac agg gca cca ccc ata ata ggg tat ttg cca ttt gaa gtt          912
Leu Asp His Arg Ala Pro Pro Ile Ile Gly Tyr Leu Pro Phe Glu Val
    290                 295                 300

ctg gga aca tca ggc tat gat tac tat cat gtg gat gac cta gaa aat          960
Leu Gly Thr Ser Gly Tyr Asp Tyr Tyr His Val Asp Asp Leu Glu Asn
305                 310                 315                 320

ttg gca aaa tgt cat gag cac tta atg caa tat ggg aaa ggc aaa tca         1008
Leu Ala Lys Cys His Glu His Leu Met Gln Tyr Gly Lys Gly Lys Ser

                325                 330                 335

tgt tat tat agg ttc ctg act aag ggg caa cag tgg att tgg ctt cag 1056
Cys Tyr Tyr Arg Phe Leu Thr Lys Gly Gln Gln Trp Ile Trp Leu Gln
            340                 345                 350

act cat tat tat atc act tac cat cag tgg aat tca agg cca gag ttt 1104
Thr His Tyr Tyr Ile Thr Tyr His Gln Trp Asn Ser Arg Pro Glu Phe
        355                 360                 365

att gtt tgt act cac act gta gta agt tat gca gaa gtt agg gct gaa 1152
Ile Val Cys Thr His Thr Val Val Ser Tyr Ala Glu Val Arg Ala Glu
    370                 375                 380

aga cga cga gaa ctt ggc att gaa gag tct ctt cct gag aca gct gct 1200
Arg Arg Arg Glu Leu Gly Ile Glu Glu Ser Leu Pro Glu Thr Ala Ala
385                 390                 395                 400

gac aaa agc caa gat tct ggg tca gat aat cgt ata aac aca gtc agt 1248
Asp Lys Ser Gln Asp Ser Gly Ser Asp Asn Arg Ile Asn Thr Val Ser
                405                 410                 415

ctc aag gaa gca ttg gaa agg ttt gat cac agc cca acc cct tct gcc 1296
Leu Lys Glu Ala Leu Glu Arg Phe Asp His Ser Pro Thr Pro Ser Ala
            420                 425                 430

tct tct cgg agt tca aga aaa tca tct cac acg gcc gtc tca gac cct 1344
Ser Ser Arg Ser Ser Arg Lys Ser Ser His Thr Ala Val Ser Asp Pro
        435                 440                 445

tcc tca aca cca acc aag atc ccg acg gat acg agc act cca ccc agg 1392
Ser Ser Thr Pro Thr Lys Ile Pro Thr Asp Thr Ser Thr Pro Pro Arg
    450                 455                 460

cag cat tta cca gct cat gag aag atg gtg caa aga agg tca tca ttt 1440
Gln His Leu Pro Ala His Glu Lys Met Val Gln Arg Arg Ser Ser Phe
465                 470                 475                 480

agt agt cag tcc ata aat tcc cag tct gtt ggt tca tca tta aca cag 1488
Ser Ser Gln Ser Ile Asn Ser Gln Ser Val Gly Ser Ser Leu Thr Gln
                485                 490                 495

cca gtg atg tct caa gct aca aat tta cca att cca caa ggc atg tcc 1536
Pro Val Met Ser Gln Ala Thr Asn Leu Pro Ile Pro Gln Gly Met Ser
            500                 505                 510

cag ttt cag ttt tca gct caa tta gga gcc atg caa cat ctg aaa gac 1584
Gln Phe Gln Phe Ser Ala Gln Leu Gly Ala Met Gln His Leu Lys Asp
        515                 520                 525

caa ttg gaa caa cgg aca cgc atg ata gaa gca aat att cat cgg caa 1632
Gln Leu Glu Gln Arg Thr Arg Met Ile Glu Ala Asn Ile His Arg Gln
    530                 535                 540

caa gaa gaa cta aga aaa att caa gaa caa ctt cag atg gtc cat ggt 1680
Gln Glu Glu Leu Arg Lys Ile Gln Glu Gln Leu Gln Met Val His Gly
545                 550                 555                 560

cag ggg ctg cag atg ttt ttg caa caa tca aat cct ggg ttg aat ttt 1728
Gln Gly Leu Gln Met Phe Leu Gln Gln Ser Asn Pro Gly Leu Asn Phe
                565                 570                 575

ggt tcc gtt caa ctt tct tct gga aat tca tct aac atc cag caa ctt 1776
Gly Ser Val Gln Leu Ser Ser Gly Asn Ser Ser Asn Ile Gln Gln Leu
            580                 585                 590

gca cct ata aat atg caa ggc caa gtt gtt cct act aac cag att caa 1824
Ala Pro Ile Asn Met Gln Gly Gln Val Val Pro Thr Asn Gln Ile Gln
        595                 600                 605

agt gga atg aat act gga cac att ggc aca act cag cac atg ata caa 1872
Ser Gly Met Asn Thr Gly His Ile Gly Thr Thr Gln His Met Ile Gln
    610                 615                 620

caa cag act tta cag agt aca tca act cag agt caa caa aat gta ctg 1920
Gln Gln Thr Leu Gln Ser Thr Ser Thr Gln Ser Gln Gln Asn Val Leu

625                 630                 635                 640

agt ggg cac agt cag caa aca tct cta ccc agt cag aca cag agc act      1968
Ser Gly His Ser Gln Gln Thr Ser Leu Pro Ser Gln Thr Gln Ser Thr
                645                 650                 655

ctt aca gcc cca ctg tat aac act atg gtg att tct cag cct gca gcc      2016
Leu Thr Ala Pro Leu Tyr Asn Thr Met Val Ile Ser Gln Pro Ala Ala
            660                 665                 670

gga agc atg gtc cag att cca tct agt atg cca caa aac agc acc cag      2064
Gly Ser Met Val Gln Ile Pro Ser Ser Met Pro Gln Asn Ser Thr Gln
        675                 680                 685

agt gct gca gta act aca ttc act cag gac agg cag ata aga ttt tct      2112
Ser Ala Ala Val Thr Thr Phe Thr Gln Asp Arg Gln Ile Arg Phe Ser
    690                 695                 700

caa ggt caa caa ctt gtg acc aaa tta gtg act gct cct gta gct tgt      2160
Gln Gly Gln Gln Leu Val Thr Lys Leu Val Thr Ala Pro Val Ala Cys
705                 710                 715                 720

ggg gca gtc atg gta cct agt act atg ctt atg ggc cag gtg gtg act      2208
Gly Ala Val Met Val Pro Ser Thr Met Leu Met Gly Gln Val Val Thr
                725                 730                 735

gca tat cct act ttt gct aca caa cag caa cag tca cag aca ttg tca      2256
Ala Tyr Pro Thr Phe Ala Thr Gln Gln Gln Ser Gln Thr Leu Ser
            740                 745                 750

gta acg cag cag cag cag cag cag agc tcc cag gag cag cag ctc act      2304
Val Thr Gln Gln Gln Gln Gln Gln Ser Ser Gln Glu Gln Gln Leu Thr
        755                 760                 765

tca gtt cag caa cca tct cag gct cag ctg acc cag cca ccg caa caa      2352
Ser Val Gln Gln Pro Ser Gln Ala Gln Leu Thr Gln Pro Pro Gln Gln
    770                 775                 780

ttt tta cag act tct agg ttg ctc cat ggg aat ccc tca act caa ctc      2400
Phe Leu Gln Thr Ser Arg Leu Leu His Gly Asn Pro Ser Thr Gln Leu
785                 790                 795                 800

att ctc tct gct gca ttt cct cta caa cag agc acc ttc cct cag tca      2448
Ile Leu Ser Ala Ala Phe Pro Leu Gln Gln Ser Thr Phe Pro Gln Ser
                805                 810                 815

cat cac cag caa cat cag tct cag caa cag cag caa ctc agc cgg cac      2496
His His Gln Gln His Gln Ser Gln Gln Gln Gln Gln Leu Ser Arg His
            820                 825                 830

agg act gac agc ttg ccc gac cct tcc aag gtt caa cca cag tag          2541
Arg Thr Asp Ser Leu Pro Asp Pro Ser Lys Val Gln Pro Gln
        835                 840                 845

<210>  8
<211>  846
<212>  PRT
<213>  Homo sapiens

<400>  8
   Met Leu Phe Thr Val Ser Cys Ser Lys Met Ser Ser Ile Val Asp Arg
   1               5                   10                  15
   Asp Asp Ser Ser Ile Phe Asp Gly Leu Val Glu Glu Asp Asp Lys Asp
               20                  25                  30
   Lys Ala Lys Arg Val Ser Arg Asn Lys Ser Glu Lys Lys Arg Arg Asp
           35                  40                  45
   Gln Phe Asn Val Leu Ile Lys Glu Leu Gly Ser Met Leu Pro Gly Asn
       50                  55                  60
   Ala Arg Lys Met Asp Lys Ser Thr Val Leu Gln Lys Ser Ile Asp Phe
   65                  70                  75                  80
   Leu Arg Lys His Lys Glu Ile Thr Ala Gln Ser Asp Ala Ser Glu Ile
                   85                  90                  95
   Arg Gln Asp Trp Lys Pro Thr Phe Leu Ser Asn Glu Glu Phe Thr Gln
               100                 105                 110
   Leu Met Leu Glu Ala Leu Asp Gly Phe Phe Leu Ala Ile Met Thr Asp
           115                 120                 125
   Gly Ser Ile Ile Tyr Val Ser Glu Ser Val Thr Ser Leu Leu Glu His
       130                 135                 140
   Leu Pro Ser Asp Leu Val Asp Gln Ser Ile Phe Asn Phe Ile Pro Glu
   145                 150                 155                 160
   Gly Glu His Ser Glu Val Tyr Lys Ile Leu Ser Thr His Leu Leu Glu
                   165                 170                 175
   Ser Asp Ser Leu Thr Pro Glu Tyr Leu Lys Ser Lys Asn Gln Leu Glu
               180                 185                 190
   Phe Cys Cys His Met Leu Arg Gly Thr Ile Asp Pro Lys Glu Pro Ser
           195                 200                 205
   Thr Tyr Glu Tyr Val Lys Phe Ile Gly Asn Phe Lys Ser Leu Asn Ser
       210                 215                 220
   Val Ser Ser Ser Ala His Asn Gly Phe Glu Gly Thr Ile Gln Arg Thr
   225                 230                 235                 240
   His Arg Pro Ser Tyr Glu Asp Arg Val Cys Phe Val Ala Thr Val Arg
                   245                 250                 255
   Leu Ala Thr Pro Gln Phe Ile Lys Glu Met Cys Thr Val Glu Glu Pro
               260                 265                 270
   Asn Glu Glu Phe Thr Ser Arg His Ser Leu Glu Trp Lys Phe Leu Phe
           275                 280                 285
   Leu Asp His Arg Ala Pro Pro Ile Ile Gly Tyr Leu Pro Phe Glu Val
       290                 295                 300
   Leu Gly Thr Ser Gly Tyr Asp Tyr Tyr His Val Asp Asp Leu Glu Asn
   305                 310                 315                 320
   Leu Ala Lys Cys His Glu His Leu Met Gln Tyr Gly Lys Gly Lys Ser
                   325                 330                 335
   Cys Tyr Tyr Arg Phe Leu Thr Lys Gly Gln Gln Trp Ile Trp Leu Gln
               340                 345                 350
   Thr His Tyr Tyr Ile Thr Tyr His Gln Trp Asn Ser Arg Pro Glu Phe
           355                 360                 365
   Ile Val Cys Thr His Thr Val Val Ser Tyr Ala Glu Val Arg Ala Glu
   370 375 380
   Arg Arg Arg Glu Leu Gly Ile Glu Glu Ser Leu Pro Glu Thr Ala Ala
   385 390 395 400
   Asp Lys Ser Gln Asp Ser Gly Ser Asp Asn Arg Ile Asn Thr Val Ser
   405 410 415
   Leu Lys Glu Ala Leu Glu Arg Phe Asp His Ser Pro Thr Pro Ser Ala
   420 425 430
   Ser Ser Arg Ser Ser Arg Lys Ser Ser His Thr Ala Val Ser Asp Pro
   435 440 445
   Ser Ser Thr Pro Thr Lys Ile Pro Thr Asp Thr Ser Thr Pro Pro Arg
   450 455 460
   Gln His Leu Pro Ala His Glu Lys Met Val Gln Arg Arg Ser Ser Phe
   465 470 475 480
   Ser Ser Gln Ser Ile Asn Ser Gln Ser Val Gly Ser Ser Leu Thr Gln
   485 490 495
   Pro Val Met Ser Gln Ala Thr Asn Leu Pro Ile Pro Gln Gly Met Ser
   500 505 510
   Gln Phe Gln Phe Ser Ala Gln Leu Gly Ala Met Gln His Leu Lys Asp
   515 520 525
   Gln Leu Glu Gln Arg Thr Arg Met Ile Glu Ala Asn Ile His Arg Gln
   530 535 540
   Gln Glu Glu Leu Arg Lys Ile Gln Glu Gln Leu Gln Met Val His Gly
   545 550 555 560
   Gln Gly Leu Gln Met Phe Leu Gln Gln Ser Asn Pro Gly Leu Asn Phe
   565 570 575
   Gly Ser Val Gln Leu Ser Ser Gly Asn Ser Ser Asn Ile Gln Gln Leu
   580 585 590
   Ala Pro Ile Asn Met Gln Gly Gln Val Val Pro Thr Asn Gln Ile Gln
   595 600 605
   Ser Gly Met Asn Thr Gly His Ile Gly Thr Thr Gln His Met Ile Gln
   610 615 620
   Gln Gln Thr Leu Gln Ser Thr Ser Thr Gln Ser Gln Gln Asn Val Leu
   625 630 635 640
   Ser Gly His Ser Gln Gln Thr Ser Leu Pro Ser Gln Thr Gln Ser Thr
   645 650 655
   Leu Thr Ala Pro Leu Tyr Asn Thr Met Val Ile Ser Gln Pro Ala Ala
   660 665 670
   Gly Ser Met Val Gln Ile Pro Ser Ser Met Pro Gln Asn Ser Thr Gln
           675                 680                 685
   Ser Ala Ala Val Thr Thr Phe Thr Gln Asp Arg Gln Ile Arg Phe Ser
       690                 695                 700
   Gln Gly Gln Gln Leu Val Thr Lys Leu Val Thr Ala Pro Val Ala Cys
   705                 710                 715                 720
   Gly Ala Val Met Val Pro Ser Thr Met Leu Met Gly Gln Val Val Thr
                   725                 730                 735
   Ala Tyr Pro Thr Phe Ala Thr Gln Gln Gln Gln Ser Gln Thr Leu Ser
               740                 745                 750
   Val Thr Gln Gln Gln Gln Gln Gln Ser Ser Gln Glu Gln Gln Leu Thr
           755                 760                 765
   Ser Val Gln Gln Pro Ser Gln Ala Gln Leu Thr Gln Pro Pro Gln Gln
       770                 775                 780
   Phe Leu Gln Thr Ser Arg Leu Leu His Gly Asn Pro Ser Thr Gln Leu
   785                 790                 795                 800
   Ile Leu Ser Ala Ala Phe Pro Leu Gln Gln Ser Thr Phe Pro Gln Ser
                   805                 810                 815
   His His Gln Gln His Gln Ser Gln Gln Gln Gln Gln Leu Ser Arg His
               820                 825                 830
   Arg Thr Asp Ser Leu Pro Asp Pro Ser Lys Val Gln Pro Gln
           835                 840                 845

<210>  9
<211>  37
<212>  DNA
<213>  Artificial

<220>
<223>  Designed oligonucleotide for use as a primer to amplify MITF-M
       gene

<400>  9
gaattcatgc tggaaatgct agaatataat cactatc                                37

<210>  10
<211>  35
<212>  DNA
<213>  Artificial

<220>
<223>  Designed oligonucleotide for use as a primer to amplify MITF-M
       gene

<400>  10
ctcgagctaa caagtgtgct ccgtctcttc catgc                                  35

<210>  11
<211>  40
<212>  DNA
<213>  Artificial

<220>
<223>  Designed oligonucleotide for use as a primer to amplify ELK4 gene

<400>  11
gaattcgcca tggacagtgc tatcaccctg tggcagttcc                              40


<210>  12
<211>  36
<212>  DNA
<213>  Artificial

<220>
<223>  Designed oligonucleotide for use as a primer to amplify ELK4 gene

<400>  12
ctcgagcatg acagtcacac ataacctttc taagac                                  36


<210>  13
<211>  36
<212>  DNA
<213>  Artificial

<220>
<223>  Designed oligonucleotide for use as a primer to amplify HLF gene

<400>  13
ggatccatgg agaaaatgtc ccgaccgctc cccctg                                  36


<210>  14
<211>  38
<212>  DNA
<213>  Artificial

<220>
<223>  Designed oligonucleotide for use as a primer to amplify HLF gene

<400>  14
ctcgagctac aggggcccgt gcctggcctc atacttgg                                38


<210>  15
<211>  32
<212>  DNA
<213>  Artificial

<220>
<223>  Designed oligonucleotide for use as a primer to amplify CLOCK
       gene

<400>  15
caagaacaac ttcagatggt ccatggtcag gg                                      32


<210>  16
<211>  31
<212>  DNA
<213>  Artificial

<220>
<223>  Designed oligonucleotide for use as a primer to amplify CLOCK
       gene

<400>  16
ctactgtggt tgaaccttgg aagggtcggg c                                       31


<210>  17
<211>  40
<212>  DNA
<213>  Artificial

<220>

<223>  Designed oligonucleotide for use as a primer to amplify ELK4 gene

<400>  17
gaattcgcca tggacagtgc tatcaccctg tggcagttcc                              40


<210>  18
<211>  36
<212>  DNA
<213>  Artificial

<220>
<223>  Designed oligonucleotide for use as a primer to amplify ELK4 gene

<400>  18
ctcgagcatg acagtcacac ataacctttc taagac                                  36


<210>  19
<211>  33
<212>  DNA
<213>  Artificial

<220>
<223>  Designed oligonucleotide for use as a primer to amplify HLF gene

<400>  19
gacagtatcg aggcttgtga tgacgaatcc tgc                                     33


<210>  20
<211>  35
<212>  DNA
<213>  Artificial

<220>
<223>  Designed oligonucleotide for use as a primer to amplify HLF gene

<400>  20
ggatggtcca tgttacaatc cttccacaat tctcc                                   35


<210>  21
<211>  7
<212>  PRT
<213>  Artificial

<220>
<223>  Partial sequence of MITF-M (SEQ ID NO:2), which is highly
       homologous to that (SEQ ID NO:22) of HLF

<400>  21

Leu Glu Asn Pro Thr Lys Tyr
1               5


<210>  22
<211>  7
<212>  PRT
<213>  Artificial

<220>
<223>  Partial sequence of HLF (SEQ ID NO:4), which is highly homologous
       to that (SEQ ID NO:21) of MITF-M

<400>  22

Leu Glu Asn Pro Leu Lys Leu
1               5


<210>  23
<211>  9
<212>  PRT
<213>  Artificial

<220>
<223>  Partial sequence of MITF-M (SEQ ID NO:2), which is highly
      homologous to that (SEQ ID NO:24) of ELK4

<400>  23

Pro Gly Ala Ser Lys Thr Ser Ser Arg
1               5


<210>  24
<211>  8
<212>  PRT
<213>  Artificial

<220>
<223>  Partial sequence of ELK4 (SEQ ID NO:6), which is highly
      homologous to that (SEQ ID NO:23) of MITF-M

<400>  24

Pro Gly Ala Lys Thr Ser Ser Arg
1               5


<210>  25
<211>  6
<212>  PRT
<213>  Artificial

<220>
<223>  Partial sequence of MITF-M (SEQ ID NO:2), which is highly
      homologous to that (SEQ ID NO:27) of CLOCK

<400>  25

Ile Lys Glu Leu Gly Thr
1               5


<210>  26
<211>  6
<212>  PRT
<213>  Artificial

<220>
<223>  Partial sequence of MITF-M (SEQ ID NO:2), which is highly
      homologous to that (SEQ ID NO:28) of CLOCK

<400>  26

Ser Ser Arg Arg Ser Ser
1               5


<210>  27
<211>  6
<212>  PRT
<213>  Artificial

<220>
<223>  Partial sequence of CLOCK (SEQ ID NO:8), which is highly
      homologous to that (SEQ ID NO:25) of MITF-M

<400>  27

Ile Lys Glu Leu Gly Ser
1               5


<210>  28
<211>  6
<212>  PRT
<213>  Artificial

<220>
<223>  Partial sequence of CLOCK (SEQ ID NO:8), which is highly
      homologous to that (SEQ ID NO:26) of MITF-M

<400>  28

Ser Ser Arg Lys Ser Ser
1               5


<210>  29
<211>  15
<212>  PRT
<213>  Artificial

<220>
<223>  Partial sequence of MITF-M (SEQ ID NO:2), which is highly
      homologous to that (SEQ ID NO:31) of HLF

<400>  29

Val Gln Thr His Leu Glu Asn Pro Thr Lys Tyr His Ile Gln Gln
1               5                   10                  15


<210>  30
<211>  6
<212>  PRT
<213>  Artificial

<220>
<223>  Sequence being homologous to the partial sequence (SEQ ID NO:29)
      of MITF-M and that (SEQ ID NO:31) of HLF


<220>
<221>  MISC_FEATURE
<222>  (5)..(5)
<223>  Xaa can be any amino acid residue.

<400>  30

Leu Glu Asn Pro Xaa Lys
1               5


<210>  31
<211>  15
<212>  PRT
<213>  Artificial

<220>
<223>  Partial sequence of HLF (SEQ ID NO:4), which is highly homologous
      to that (SEQ ID NO:29) of MITF-M

<400>  31

Leu Arg Ser Leu Leu Glu Asn Pro Leu Lys Leu Pro Leu His His
1               5                   10                  15


<210>  32
<211>  9
<212>  PRT
<213>  Artificial

<220>
<223>  Sequence being homologous to the partial sequence (SEQ ID NO:23)
      of MITF-M and that (SEQ ID NO:24) of ELK4


<220>
<221>  MISC_FEATURE
<222>  (4)..(4)
<223>  Xaa can be any amino acid residue or can be being deleted.

<400>  32

Pro Gly Ala Xaa Lys Thr Ser Ser Arg
1               5


<210>  33
<211>  56
<212>  PRT
<213>  Artificial

<220>
<223>  Partial sequence of MITF-M (SEQ ID NO:2), which is highly
       homologous to that (SEQ ID NO:35) of CLOCK

<400>  33

Lys Glu Arg Gln Lys Lys Asp Asn His Asn Leu Ile Glu Arg Arg Arg
1               5                   10                  15


Arg Phe Asn Ile Asn Asp Arg Ile Lys Glu Leu Gly Thr Leu Ile Pro
            20                  25                  30


Lys Ser Asn Asp Pro Asp Met Arg Trp Asn Lys Gly Thr Ile Leu Lys
        35                  40                  45


Ala Ser Val Asp Tyr Ile Arg Lys
    50                  55


<210>  34
<211>  56
<212>  PRT
<213>  Artificial

<220>
<223>  Sequence being homologous to the partial sequence (SEQ ID NO:33)
       of MITF-M and that (SEQ ID NO:35) of CLOCK


<220>
<221>  MISC_FEATURE
<222>  (2)..(2)
<223>  Xaa can be any amino acid residue.

<220>
<221>  MISC_FEATURE
<222>  (3)..(3)
<223>  Xaa can be any amino acid residue.

<220>
<221>  MISC_FEATURE
<222>  (4)..(4)
<223>  Xaa can be any amino acid residue.

<220>
<221>  MISC_FEATURE
<222>  (6)..(6)
<223>  Xaa can be any amino acid residue.

<220>
<221>  MISC_FEATURE
<222>  (7)..(7)
<223>  Xaa can be any amino acid residue.

<220>
<221>  MISC_FEATURE
<222>  (8)..(8)
<223>  Xaa can be any amino acid residue.

<220>
<221>  MISC_FEATURE
<222>  (9)..(9)

<223>  Xaa can be any amino acid residue.

<220>
<221>  MISC_FEATURE
<222>  (10)..(10)
<223>  Xaa can be any amino acid residue.

<220>
<221>  MISC_FEATURE
<222>  (11)..(11)
<223>  Xaa can be any amino acid residue.

<220>
<221>  MISC_FEATURE
<222>  (12)..(12)
<223>  Xaa can be any amino acid residue.

<220>
<221>  MISC_FEATURE
<222>  (14)..(14)
<223>  Xaa can be any amino acid residue.

<220>
<221>  MISC_FEATURE
<222>  (15)..(15)
<223>  Xaa can be any amino acid residue.

<220>
<221>  MISC_FEATURE
<222>  (18)..(18)
<223>  Xaa can be any amino acid residue.

<220>
<221>  MISC_FEATURE
<222>  (19)..(19)
<223>  Xaa can be any amino acid residue.

<220>
<221>  MISC_FEATURE
<222>  (20)..(20)
<223>  Xaa can be any amino acid residue.

<220>
<221>  MISC_FEATURE
<222>  (22)..(22)
<223>  Xaa can be any amino acid residue.

<220>
<221>  MISC_FEATURE
<222>  (23)..(23)
<223>  Xaa can be any amino acid residue.

<220>
<221>  MISC_FEATURE
<222>  (29)..(29)
<223>  Xaa can be any amino acid residue.

<220>
<221>  MISC_FEATURE
<222>  (30)..(30)
<223>  Xaa can be any amino acid residue.

<220>
<221>  MISC_FEATURE
<222>  (31)..(31)
<223>  Xaa can be any amino acid residue.

<220>
<221>  MISC_FEATURE
<222>  (33)..(33)
<223>  Xaa can be any amino acid residue or can be being deleted.

<220>
<221>  MISC_FEATURE
<222>  (34)..(34)
<223>  Xaa can be any amino acid residue.

<220>
<221>  MISC_FEATURE
<222>  (36)..(36)
<223>  Xaa can be any amino acid residue.

<220>
<221>  MISC_FEATURE
<222>  (37)..(37)
<223>  Xaa can be any amino acid residue.

<220>
<221>  MISC_FEATURE
<222>  (38)..(38)
<223>  Xaa can be any amino acid residue.

<220>
<221>  MISC_FEATURE
<222>  (40)..(40)
<223>  Xaa can be any amino acid residue or can be being deleted.

<220>
<221>  MISC_FEATURE
<222>  (41)..(41)
<223>  Xaa can be any amino acid residue or can be being deleted.

<220>
<221>  MISC_FEATURE
<222>  (42)..(42)
<223>  Xaa can be any amino acid residue.

<220>
<221>  MISC_FEATURE
<222>  (44)..(44)
<223>  Xaa can be any amino acid residue.

<220>
<221>  MISC_FEATURE
<222>  (46)..(46)
<223>  Xaa can be any amino acid residue.

<220>
<221>  MISC_FEATURE
<222>  (48)..(48)
<223>  Xaa can be any amino acid residue.

<220>
<221>  MISC_FEATURE
<222>  (49)..(49)
<223>  Xaa can be any amino acid residue.

<220>
<221>  MISC_FEATURE
<222>  (51)..(51)
<223>  Xaa can be any amino acid residue.

<220>
<221>  MISC_FEATURE
<222>  (53)..(53)
<223>  Xaa can be any amino acid residue.

<220>
<221>  MISC_FEATURE
<222>  (54)..(54)
<223>  Xaa can be any amino acid residue.

<400>  34

Lys Xaa Xaa Xaa Lys Xaa Xaa Xaa Xaa Xaa Xaa Xaa Glu Xaa Xaa Arg
1               5                   10                  15

Arg Xaa Xaa Xaa Asn Xaa Xaa Ile Lys Glu Leu Gly Xaa Xaa Xaa Pro
            20                  25                  30

Xaa Xaa Asn Xaa Xaa Xaa Met Xaa Xaa Xaa Lys Xaa Thr Xaa Leu Xaa
        35                  40                  45

Xaa Ser Xaa Asp Xaa Xaa Arg Lys
    50                  55

<210>  35
<211>  53
<212>  PRT
<213>  Artificial

<220>
<223>  Partial sequence of CLOCK (SEQ ID NO:8), which is highly
       homologous to that (SEQ ID NO:33) of MITF-M

<400>  35

Lys Asp Lys Ala Lys Arg Val Ser Arg Asn Lys Ser Glu Lys Lys Arg
1               5                   10                  15

Arg Asp Gln Phe Asn Val Leu Ile Lys Glu Leu Gly Ser Met Leu Pro
            20                  25                  30

Gly Asn Ala Arg Lys Met Asp Lys Ser Thr Val Leu Gln Lys Ser Ile
        35                  40                  45

Asp Phe Leu Arg Lys
    50

<210>  36
<211>  15
<212>  PRT
<213>  Artificial

<220>
<223>  Partial sequence of MITF-M (SEQ ID NO:2), which is highly
       homologous to that (SEQ ID NO:38) of CLOCK

<400>  36

Ser Val Ser Pro Gly Ala Ser Lys Thr Ser Ser Arg Arg Ser Ser
1               5                   10                  15

<210>  37
<211>  15
<212>  PRT
<213>  Artificial

<220>
<223>  Sequence being homologous to the partial sequence (SEQ ID NO:36)
       of MITF-M and that (SEQ ID NO:38) of CLOCK

<220>
<221>  MISC_FEATURE
<222>  (2)..(2)
<223>  Xaa can be any amino acid residue.

<220>
<221>  MISC_FEATURE
<222>  (3)..(3)
<223>  Xaa can be any amino acid residue.

<220>
<221>  MISC_FEATURE
<222>  (5)..(5)
<223>  Xaa can be any amino acid residue.

<220>

<221>  MISC_FEATURE
<222>  (8)..(8)
<223>  Xaa can be any amino acid residue.

<220>
<221>  MISC_FEATURE
<222>  (9)..(9)
<223>  Xaa can be any amino acid residue.

<220>
<221>  MISC_FEATURE
<222>  (13)..(13)
<223>  Xaa can be any amino acid residue.

<400>  37

Ser Xaa Xaa Pro Xaa Ala Ser Xaa Xaa Ser Ser Arg Xaa Ser Ser
1               5                   10                  15


<210>  38
<211>  15
<212>  PRT
<213>  Artificial

<220>
<223>  Partial sequence of CLOCK (SEQ ID NO:8), which is highly
       homologous to that (SEQ ID NO:36) of MITF-M

<400>  38

Ser Pro Thr Pro Ser Ala Ser Ser Arg Ser Ser Arg Lys Ser Ser
1               5                   10                  15

## Description

### TECHNICAL FIELD

The present invention relates to a method of inhibiting production of BCL2 (B-cell chronic lymphatic leukemia/lymphoma 2) gene product and an agent for inhibiting the same, which inhibits binding of protein selected from a group consisting of HLF (hepatic leukemia factor), ELK4 (ETS (erythroblast transformation specific)-domain protein Elk-4) and CLOCK (circadian locomoter output cycles kaput protein) to MITF-M (microphthalmia-associated transcription factor isoform MITF-M). Further, the present invention relates to a method of inhibiting production of BCL2 gene product, comprising using an inhibitor of the aforementioned binding, and to an agent for inhibiting the production of the gene product, containing the inhibitor. Furthermore, the present invention relates to a method of inducing cell death of melanoma cells and an agent for inducing the same, which inhibits the aforementioned binding. Further, the present invention relates to a method of inducing cell death of melanoma cells, comprising using an inhibitor of the aforementioned binding, and to an agent for inducing cell death of melanoma cells, containing the inhibitor. Furthermore, the present invention relates to a method of identifying a compound that inhibits the aforementioned binding. Further, the present invention relates to a method of identifying a compound that inhibits the production of BCL2 gene product. Furthermore, the present invention relates to a method of identifying a compound that induces cell death of melanoma cells. Further, the present invention relates to an inhibitor of the aforementioned binding. Furthermore, the present invention relates to a method of treating and/or preventing diseases accompanied by enhanced production of BCL2 gene product, such as melanoma, and an agent for treating and/or preventing the same, which inhibits the aforementioned binding. Further, the present invention relates to a method of treating and/or preventing diseases accompanied by enhanced production of BCL2 gene product, such as melanoma, comprising using an inhibitor of the aforementioned binding and/or an agent for inducing the aforementioned cell death, and to an agent for treating and/or preventing the diseases, containing the inhibitor and/or the agent. Furthermore, the present invention relates to a method of treating melanoma, comprising using at least one or more kind of agents for treating melanoma that are selected from agents for treating melanoma which inhibit the aforementioned binding, together with dacarbazine (DTIC). Further, the present invention relates to a reagent kit, containing at least one member of protein selected from a group consisting of HLF, ELK4 and CLOCK, a polynucleotide encoding the protein, a recombinant vector containing the polynucleotide and a transformant containing the recombinant vector; and at lease one member of MITF-M, a polynucleotide encoding MITF-M, a recombinant vector containing the polynucleotide and a transformant containing the recombinant vector.

### BACKGROUND OF INVENTION

Melanoma (malignant melanoma) is a malignant tumor resulted from transformation of melanocyte (melanin pigment producing cell). Melanoma has a tendency to develop early metastasis and is resistant to chemotherapy and to radiation therapy: therefore, it has been understood to be a tumor with high malignant potential (Non-patent References 1 and 2).

Enhanced expression of BCL2 gene in melanoma has been considered to be a possible cause of resistance of melanoma to chemotherapy and to radiation therapy (Non-patent References 3-5). BCL2, a gene product of BCL2 gene, is a protein that shows an anti-apoptotic activity through regulating mitochondrial membrane (Non-patent Reference 6). In other words, BCL2 prevents apoptotic cell death.

Specifically, it has been reported that the expression of BCL2 gene was enhanced by chemotherapy and that the expression of BCL2 gene was further enhanced in a lesion being resistant to chemotherapy (Non-patent Reference 5). In addition, it has been reported that treatment of melanoma with a BCL2 gene antisense oligonucleotide to decrease an amount of BCL2 resulted in enhanced apoptosis of melanoma and in increased sensitivity of melanoma to chemotherapy (Non-patent References 3 and 4). The expression of BCL2 gene or involvement of BCL2 gene has been reported also for other cancers than melanoma (Non-patent References 7-16).

MITF-M is an isoform of MITF (microphfhalmia-associated transcription factor) and is known to be a transcription factor essential for melanocyte development and survival (Non-patent Reference 17). A MITF-M encoding gene is expressed specifically in melanocyte and melanoma (Non-patent Reference 18). It has been revealed that MTTF-M positively regulates BCL2 gene expression in melanocyte and melanoma (Non-patent Reference 19).

HLF is a transcription factor belonging to PAR (proline and acidic amino acid-rich) subfamily and has been reported to be highly expressed in liver. HLF forms, in cells, a homodimer or a heterodimer with the other transcription factor belonging to PAR subfamily. Further, it is reported that a fusion protein of HLF and E2A due to translocation is present in B cells derived from patients with acute B-lineage leukemia.

ELK4 is a transcription factor having ETS (erythroblast transformation specific) domain and is known to be expressed in variety of human tissues. ELK4 forms, in cells, a complex with a dimer of SRF(c-fos serum response element-binding transcription factor) that is a transcription factor. The complex binds to SRE (serum response element) present in a promoter region of c-fos gene, and activates transcription of c-fos gene.

CLOCK is a transcription factor having bHLH (basic helix-loop-helix) domain and is known to relate to a circadian rhythm. CLOCK forms, in cells, a heterodimer with BMAL1 (brain and muscle arylhydrocarbon receptor nuclear translocator-like protein 1) that also relates to a circadian rhythm, and regulates transcription of Per1 gene, a member of period genes. In addition, CLOCK is known to form a heterodimer with the other transcription factor belonging to bHLH family.

However, HLF, ELK4 and CLOCK have not yet been shown to relate to melanoma, MITF-M or BCL2.

The References cited in the specification are listed as follows:
Patent Reference 1: International Publication No. WO 01/67299 pamphlet.
Non-patent Reference 1: KAGAKURYOUHOU NO RYOUIKI (Antibiotics & Chemotherapy), 2003, S-1, Vol. 19, p. 224-231.
Non-patent Reference 2: Oncogene, 2003, Vol. 22, p. 3138-3151.
Non-patent Reference 3: Nature Medicine, 1998, Vol. 4, p. 232-234.
Non-patent Reference 4: The Lancet, 2000, Vol. 356, p. 1728-1733.
Non-patent Reference 5: Cancer Immunology, Immunotherapy, 2003, Vol. 52, p. 249-254.
Non-patent Reference 6: SAISHINIGAKU, 2002, Vol. 15, p. 2447-2453.
Non-patent Reference 7: Cell, 1986, Vol. 47, p. 19-28.
Non-patent Reference 8: Cancer Research, 1995, Vol. 55, p. 237-241.
Non-patent Reference 9: Cancer Research, 1995, Vol. 55, p. 4438-4445.
Non-patent Reference 1 0: Oncogene, 1998, Vol. 16, p. 933-943.
Non-patent Reference 11: International Journal of Cancer, 1997, Vol. 73, p. 38-41.
Non-patent Reference 12: Oncogene, 2002, Vol. 21, p. 7611-7618.
Non-patent Reference 13: International Journal of Cancer, 1995, Vol. 60, p. 54-60.
Non-patent Reference 14: Human Pathology, 1998, Vol. 29, p. 965-971.
Non-patent Reference 15: Seminars in Oncology, 1999, Vol. 26, p. 112-116.
Non-patent Reference 16: Blood, 1997, Vol. 89, p. 601-609.
Non-patent Reference 17: SEIKAGAKU (Journal of Biochemistry), 2003, Vol. 75, p. 1444-1448.
Non-patent Reference 18: Oncogene, 2003, Vol. 22, p. 3035-3041.
Non-patent Reference 19: Cell, 2002, Vol. 109, p. 707-718.
Non-patent Reference 20: Ulmer K.M., Science, 1983, Vo1.219, p.666-671.
Non-patent Reference 21: PEPUTIDO GOUSEI, Maruzen Co., Ltd., 1975.
Non-patent Reference 22: Peptide Synthesis, Interscience, New York, 1996.
Non-patent Reference 23: Muramatsu Masami., Ed., Labomanual Genetic Engineering, 1988, Maruzen Co., Ltd.
Non-patent Reference 24: Ehrlich, H. A., Ed, PCR Technology. Principles and Applications for DNA Amplification, 1989, Stockton Press.
Non-patent Reference 25: Madin, K., et al., Proceedings of The National Academy of Sciences of The United States of America, 2000, Vol. 97, p. 559-564.

### DISCLOSURE OF THE INVENTION

### (PROBLEMS TO BE SOLVED BY THE INVENTION)

The present inventors believe that elucidating a regulation mechanism of BCL2 gene expression by MITF-M and reducing the BCL2 gene expression by means of regulating the mechanism leads to elucidation of diseases accompanied by enhanced production of BCL2 gene product, such as melanoma and the like, as well as treatment and/or prevention of the same.

An object of the present invention is to find out a protein that relates to regulation of transcription activating activity of MITF-M, and to reduce the transcription activating activity of MITF-M by means of regulating the protein and an effect of the protein on MITF-M, thereby to provide a means for inhibiting production of BCL2 gene product. Further, an object of the present invention is to provide a means for treating and/or preventing diseases accompanied by enhanced production of BCL2 gene product, such as melanoma, by means of inhibiting production of BCL2 gene product.

### (MEANS FOR SOLVING THE PROBLEM)

The present inventors have concentrated their efforts to meet the aforementioned objects, and predicted in-*silico* that MITF-M interacts with HLF, ELK4, or CLOCK. Then, it was proved in in-vitro binding assay that MITF-M bound to HLF, ELK4 or CLOCK. It was also proved in a reporter assay using BCL2 gene promoter that expression of reporter gene was enhanced in a case where any one selected from a group consisting of HLF gene, ELK4 gene and CLOCK gene was co-expressed with MITF-M, compared to in a case where only MITF-M gene was expressed.

The present inventors believe from these findings that MITF-M binds to HLF, ELK4 or CLOCK resulting in enhanced transcription activating activity of MITF-M, which leads to an enhancement of BCL2 gene expression since MITF-M affects BCL2 gene as a transcription factor. Therefore, the present inventors believe that it is possible to reduce the transcription activating activity of MITF-M by inhibiting binding of MITF-M to protein selected from a group consisting of HLF, ELK4 and CLOCK, and consequently to inhibit the enhancement of BCL2 gene expression. BCL2, a gene product of BCL2 gene, is a protein having an anti-apoptotic activity. It has been reported that the expression of BCL2 gene relates to cancers such as melanoma. The present inventors believe from these facts that it is possible to induce cell death by inhibiting the expression of BCL2 gene, and consequently to prevent and/or treat cancers such as melanoma.

The present invention has been achieved based on these findings.

In various embodiments, the present invention relates to a method of inhibiting production of BCL2 gene product, comprising inhibiting binding of protein selected from a group consisting of (i) HLF, (ii) ELK4, and (iii) CLOCK to MITF-M.

The present invention further relates to a method of inhibiting production of BCL2 gene product, comprising using an agent for inhibiting binding of protein selected from a group consisting of (i) HLF, (ii) ELK4, and (iii) CLOCK to MITF-M.

The present invention still further relates to a method of inducing cell death of melanoma cells, comprising inhibiting binding of protein selected from a group consisting of (i) HLF, (ii) ELK4, and (iii) CLOCK to MITF-M.

The present invention also relates to a method of inducing cell death of melanoma cells, comprising using an agent for inhibiting binding of protein selected from a group consisting of (i) HLF, (ii) ELK4, and (iii) CLOCK to MITF-M.

The present invention further relates to a method of identifying a compound that inhibits binding of a protein (protein A) selected from a group consisting of (i) HLF, (ii) ELK4, and (iii) CLOCK to MITF-M, comprising contacting a compound with protein A and/or MITF-M under conditions that allow for interaction of the compound with protein A and/or MITF-M, employing a system using a signal and/or marker generated by binding of protein A to MITF-M; and detecting presence or absence or change of the signal and/or marker to determine whether the compound inhibits the binding of protein A to MTRF-M.

The present invention still further relates to a method of identifying a compound that inhibits production of BCL2 gene product, comprising contacting a compound with a protein (protein A) selected from a group consisting of (i) HLF, (ii) ELK4, and (iii) CLOCK and/or with MITF-M under conditions that allow for binding of protein A to MITF-M and for interaction of the compound with protein A and/or MITF-M, and determining whether the compound inhibits production of BCL2 gene product.

The present invention also relates to a method of identifying a compound that induces cell death of melanoma cells, comprising contacting a compound with a protein (protein A) selected from a group consisting of (i) HLF, (ii) ELK4, and (iii) CLOCK and/or with MITF-M under conditions that allow for binding of protein A to MITF-M and for interaction of the compound with protein A and/or MITF-M, and determining whether the compound induces cell death of melanoma cells.

The present invention further relates to an agent for inhibiting binding of protein selected from a group consisting of (i) HLF, (ii) ELK4, and (iii) CLOCK to MITF-M.

The present invention still further relates to an agent for inhibiting production of BCL2 gene product, which inhibits binding of protein selected from a group consisting of (i) HLF, (ii) ELK4, and (iii) CLOCK to MITF-M.

The present invention also relates to an agent for inhibiting production of BCL2 gene product, containing an effective amount of an agent for inhibiting binding of protein selected from a group consisting of (i) HLF, (ii) ELK4, and (iii) CLOCK to MITF-M.

The present invention further relates to an agent for inducing cell death of melanoma, which inhibits binding of protein selected from a group consisting of (i) HLF, (ii) ELK4, and (iii) CLOCK to MITF-M.

The present invention still further relates to an agent for inducing cell death of melanoma, containing an effective amount of an agent for inhibiting binding of protein selected from a group consisting of (i) HLF, (ii) ELK4, and (iii) CLOCK to MITF-M.

The present invention also relates to an agent for preventing and/or treating a disease accompanied by enhanced production of BCL2 gene product, which inhibits binding of protein selected from a group consisting of (i) HLF, (ii) ELK4, and (iii) CLOCK to MITF-M.

The present invention further relates to an agent for preventing and/or treating a disease accompanied by enhanced production of BCL2 gene product, containing an effective amount of an agent for inhibiting binding of protein selected from a group consisting of (i) HLF, (ii) ELK4, and (iii) CLOCK to MITF-M.

The present invention still further relates to an agent for preventing and/or treating a disease accompanied by enhanced production of BCL2 gene product, containing the aforementioned inhibiting agent and/or the aforementioned agent for inducing cell death.

The present invention also relates to the aforementioned preventing and/or treating agent, wherein the disease accompanied by enhanced production of BCL2 gene product is melanoma.

The present invention further relates to a method of preventing and/or treating a disease accompanied by enhanced production of BCL2 gene product, comprising inhibiting binding of protein selected from a group consisting of (i) HLF, (ii) ELK4, and (iii) CLOCK to MITF-M.

The present invention still further relates to a method of preventing and/or treating a disease accompanied by enhanced production of BCL2 gene product, comprising using an agent for inhibiting binding of protein selected from a group consisting of (i) HLF, (ii) ELK4, and (iii) CLOCK to MITF-M.

The present invention also relates to a method of preventing and/or treating a disease accompanied by enhanced production of BCL2 gene product, comprising using the aforementioned inhibiting agent and/or the aforementioned agent for inducing cell death.

The present invention further relates to the aforementioned preventing and/or treating method, wherein the disease accompanied by enhanced production of BCL2 gene product is melanoma.

The present invention still further relates to a method of treating melanoma, comprising using the aforementioned treating agent together with dacarbazine (DTIC).

The present invention also relates to a reagent kit, containing at least one member of a protein (protein A) selected from a group consisting of (i) HLF, (ii) ELK4, and (iii) CLOCK, a polynucleotide encoding the protein A, a recombinant vector containing the polynucleotide and a transformant containing the recombinant vector; and at lease one member of MITF-M, a polynucleotide encoding MTTF-M, a recombinant vector containing the polynucleotide and a transformant containing the recombinant vector.

### ADVANTAGEOUS EFFECT OF THE INVENTION

The present invention comprises inhibiting binding of protein selected from HLF, ELK4 and CLOCK to MITF-M. The present invention makes it possible to inhibit an enhancement of transcription activating activity of MITF-M due to protein selected from HLF, ELK4 and CLOCK, and consequently to inhibit production of BCL2 gene product since MITF-M affects the gene as a transcription factor.

A gene product of BCL2 gene is a protein having an anti-apoptotic activity. Therefore, it is possible to reduce the anti-apoptotic activity and to induce apoptosis of cells by inhibiting the production of BCL2 gene product. It has been considered that enhanced expression of BCL2 gene can be a cause of resistance of melanoma to chemotherapy and to radiation therapy. Therefore, it is possible to increase sensitivity of melanoma to chemotherapy and to radiation therapy by inhibiting the production of BCL2 gene product.

Thus, the present invention can be utilized in treatment and/or prevention of diseases accompanied by enhanced production of BCL2 gene product. Specifically, for example, it is possible to induce apoptosis of melanoma, or to increase sensitivity of melanoma to chemotherapy and to radiation therapy.

### BRIEF DESCRIPTION OF THE DRAWINGS

Fig. 1-A shows results of *in silico* prediction that MITF-M interacts with HLF. The region is shown that exhibited a high score as a result of local alignment between MITF-M and HLF. The amino acid sequences are represented in the single-letter code. A sequence shown between partial sequences of MITF-M and HLF indicates common amino acids and their positions in the partial sequences of MITF-M and HLF. The number in the figure indicates position of the N-terminal amino acid of each region shown in the figure in the amino acid sequence of MITF-M or HLF. (Example 1)

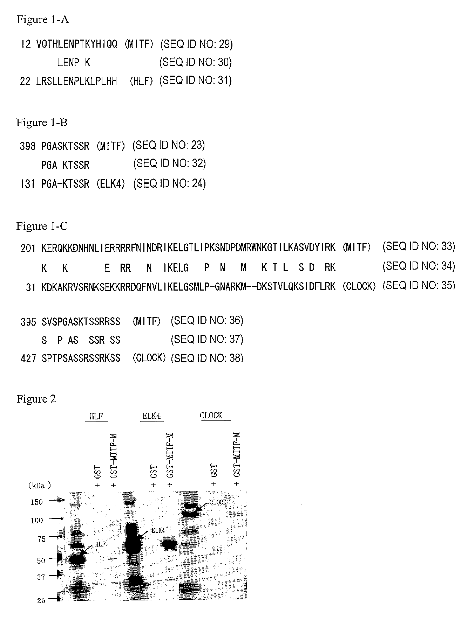

Fig. 1-B shows results of *in silico* prediction that MITF-M interacts with ELK4. The region is shown that exhibited a high score as a result of local alignment between MITF-M and ELK4. The amino acid sequences are represented in the single-letter code. A sequence shown between partial sequences of MITF-M and ELK4 indicates common amino acids and their positions in the partial sequences of MITF-M and ELK4. The number in the figure indicates position of the N-terminal amino acid of each region shown in the figure in the amino acid sequence of MITF-M or ELK4. (Example 1)
Fig. 1-C shows results of *in silico* prediction that MITF-M interacts with CLOCK. The region is shown that exhibited a high score as a result of local alignment between MITF-M and CLOCK. The amino acid sequences are represented in the single-letter code. A sequence shown between partial sequences of MITF-M and CLOCK indicates common amino acids and their positions in the partial sequences of MITF-M and CLOCK. The number in the figure indicates position of the N-terminal amino acid of each region shown in the figure in the amino acid sequence of MITF-M or CLOCK. (Example 1)

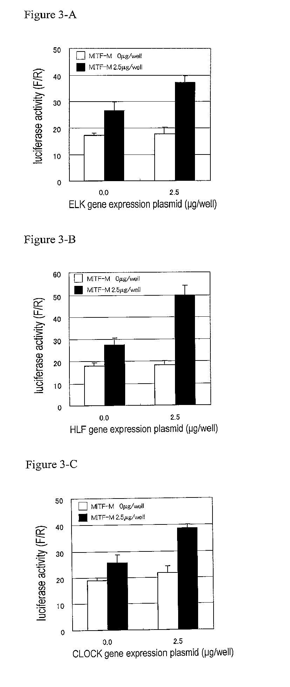

Fig. 2 shows that MITF-M binds to HLF, ELK4 or CLOCK. In an experimental system which comprises bringing HLF, ELK4 or CLOCK into reaction with GST-MITF-M (MITF-M prepared as a fusion protein with glutathione S-transferase (GST)) and subsequently detecting a protein that binds to GST-MITF-M by means of an electrophoresis, a band of a protein bound to GST-MITF-M was detected at a position corresponding to a molecular weight of HLF, ELK4 or CLOCK. Such a band was not detected in reaction of HLF, ELK4 or CLOCK with GST. (Example 2)
Fig. 3-A shows that in a reporter assay using BCL2 gene promoter, luciferase activity was approximately 1.4 fold increased in cells transfected with a MITF-M gene expression plasmid together with an ELK4 gene expression plasmid compared to in cells that was not transfected with the ELK4 expression plasmid (black column). On the other hand, luciferase activity was not changed in cells that were not transfected with the MITF-M gene expression plasmid even if the cells were transfected with the ELK4 gene expression (white column). (Example 3)
Fig. 3-B shows that in a reporter assay using BCL2 gene promoter, luciferase activity was approximately 1.8 fold increased in cells transfected with a MITF-M gene expression plasmid together with a HLF gene expression plasmid compared to in cells that was not transfected with the HLF expression plasmid (black column). On the other hand, luciferase activity was not changed in cells that were not transfected with the MITF-M gene expression plasmid even if the cells were transfected with the HLF gene expression (white column). (Example 3)
Fig. 3-C shows that in a reporter assay using BCL2 gene promoter, luciferase activity was approximately 1.5 fold increased in cells transfected with a MITF-M gene expression plasmid together with a CLOCK gene expression plasmid compared to in cells that was not transfected with the CLOCK expression plasmid (black column). On the other hand, luciferase activity was not changed in cells that were not transfected with the MITF-M gene expression plasmid even if the cells were transfected with the CLOCK gene expression (white column). (Example 3)

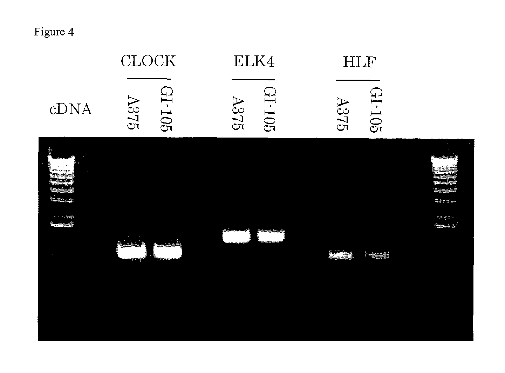

Fig. 4 shows that expression of HLF gene, ELK4 gene and CLOCK gene was detected in both cDNAs derived from melanoma cells, A375 and GI-105. (Example 4)

### DETAILED DESCRIPTION OF THE INVENTION

Embodiments of the present invention are explained in further detail below.
In the present specification, the term "protein" may be used as a generic term which includes the followings: an isolated or a synthetic full-length protein; an isolated or a synthetic full-length polypeptide; and an isolated or a synthetic full-length oligopeptide. A protein, a polypeptide or an oligopeptide used herein comprises two or more amino acids that are bound to each other by peptide bond or modified peptide bond. Herein after, an amino acid may be represented by a single letter or by three letters.

In the present invention, the interaction of MITF-M with HLF, ELK4 or CLOCK was predicted *in-silico* according to the method described in the Patent Reference 1. Then, it was proved in in-vitro binding assay that MITF-M bound to HLF, ELK4 or CLOCK. Furthermore, it was found at first time that the binding of MITF-M to HLF, ELK4 or CLOCK leads to an enhancement of transcriptional activity of BCL2 gene promoter to which MITF-M affects as a transcription factor.

The present inventors believe from these findings that MITF-M binds to HLF, ELK4 or CLOCK resulting in enhanced transcription activating activity of MITF-M, which leads to an enhancement of BCL2 gene expression since MITF-M affects BCL2 gene as a transcription factor.

A gene product of BCL2 gene is a protein having an anti-apoptotic activity. It has been reported that the expression of BCL2 gene relates to cancers such as melanoma. For example, it has been reported that MITF-M positively regulates BCL2 gene expression in melanocyte and melanoma (Non-patent Reference 19). These facts indicate that inhibition of the expression of BCL2 gene can allow for induction of cell death. It is possible to reduce or decrease the amount of production of BCL2 gene product by inhibiting binding of MITF-M to protein selected from a group consisting of HLF, ELK4 and CLOCK, and consequently to induce cell death of cells such as melanoma cells. Furthermore, it is possible to carry out treatment and/or prevention of diseases accompanied by the enhanced production of BCL2 gene product, such as melanoma.

Enhanced expression of BCL2 gene has been reported to be a cause of resistance to chemotherapy of, for example, melanoma (Non-patent References 3-5). In addition, it has been reported that treatment of melanoma with a BCL2 gene antisense oligonucleotide to decrease an amount of BCL2 resulted in enhanced apoptosis of melanoma and in increased sensitivity of melanoma to chemotherapy with dacarbazine (DTIC) (Non-patent References 3 and 4). Therefore, it is possible to increase the sensitivity of melanoma to chemotherapy by inhibiting binding of protein selected from HLF, ELK4 and CLOCK to MITF-M and thereby inhibiting the production of BCL2 gene product. For example, it is possible to increase the sensitivity of melanoma to dacarbazine.

The present invention was achieved based on these findings. One aspect of the present invention relates to a method of and an agent for inhibiting production of BCL2 gene product, which inhibit binding of protein selected from HLF, ELK4 and CLOCK to MITF-M.

Another aspect of the present invention relates to a method of and an agent for inducing cell death of melanoma cells, which inhibit binding of protein selected from HLF, ELK4 and CLOCK to MITF-M.

Further aspect of the present invention relates to a method of and an agent for treating and/or preventing diseases accompanied by enhanced production of BCL2 gene product such as melanoma, which inhibit binding ofprotein selected from HLF, ELK4 and CLOCK to MITF-M.

In addition, the present invention can provide a method of and an agent for increasing sensitivity of melanoma to melanoma drugs, which inhibit binding of protein selected from HLF, ELK4 and CLOCK to MITF-M.

The agent for inhibiting production of BCL2 gene product, the agent for inducing cell death of melanoma cells, and the agent for treating and/or preventing diseases accompanied by enhanced production of BCL2 gene product, which are provided in the present invention, can increase sensitivity of melanoma to melanoma drugs, and therefore can provide a significant effect in melanoma treatment when using in combination with melanoma drugs compared to when using melanoma drugs only. Thus, the present invention can provide an agent for treating melanoma, which comprises the agent for inhibiting production of BCL2 gene product, the agent for inducing cell death of melanoma cells, and the agent for treating and/or preventing diseases accompanied by enhanced production of BCL2 gene product, which are provided in the present invention, in combination with melanoma drugs. In addition, the present invention can provide a method of treating melanoma, comprising using the agent for inhibiting production of BCL2 gene product, the agent for inducing cell death of melanoma cells, and the agent for treating and/or preventing diseases accompanied by enhanced production of BCL2 gene product, which are provided in the present invention, in combination with melanoma drugs. As such melanoma drugs, any well known melanoma drugs can be used. The melanoma drugs can be single medication, or combination medications comprising more than two melanoma drugs. It is preferably exemplified by well known melanoma drugs to which sensitivity of melanoma increases with reduction of BCL2 amount in the melanoma. It is more preferably exemplified by such melanoma drugs as those to which sensitivity of melanoma increases with reduction of BCL2 amount in the melanoma where the reduction of BCL2 amount is given by inhibition of the binding of protein selected from HLF, ELK4 and CLOCK to MITF-M. Dacarbazine (DTIC) can be most preferably exemplified.

MITF-M, HLF, ELK4 and CLOCK can be any of those proteins derived from tissues, cells or the like of animals, preferably mammals, such as humans, mice, rats, rabbits, cows, monkeys and the like. Preferable examples include proteins derived from melanoma, liver, brain or cells thereof originated in humans. Specifically, a gene encoding MITF-M and a protein encoded by the gene can be preferably exemplified by a polynucleotide and a protein originated in humans respectively represented by sequences set forth in SEQ ID NO: 1 and SEQ ID NO: 2. A gene encoding HLF and a protein encoded by the gene can be preferably exemplified by a polynucleotide and a protein originated in humans respectively represented by sequences set forth in SEQ ID NO: 3 and SEQ ID NO: 4. A gene encoding ELK4 and a protein encoded by the gene can be preferably exemplified by a polynucleotide and a protein originated in humans respectively represented by sequences set forth in SEQ ID NO: 5 and SEQ ID NO: 6. A gene encoding CLOCK and a protein encoded by the gene can be preferably exemplified by a polynucleotide and a protein originated in humans respectively represented by sequences set forth in SEQ ID NO: 7 and SEQ ID NO: 8. MITF-M, HLF, ELK4, CLOCK and genes encoding thereof are not limited to the proteins and polynucleotides respectively represented by the aforementioned sequences, and can be proteins and polynucleotides with a mutation of one or several amino acids or nucleotide in the proteins and polynucleotides represented by the aforementioned sequences as long as those are proteins having a function of MITF-M, HLF, ELK4, CLOCK generally known and polynucleotides encoding the proteins. Further, those can be proteins and polynucleotides prepared by introducing a mutation of one or several amino acids or nucleotide in the proteins and polynucleotides represented by the aforementioned sequences in order to increase or decrease the function.

The phrase "protein selected from HLF, ELK4 and CLOCK" refers to one or more proteins selected from HLF, ELK4 and CLOCK, and preferably refers to HLF, ELK4 or CLOCK.

The phrase "binding of protein selected from HLF, ELK4 and CLOCK to MITF-M" refers to interaction of the protein with MITF-M so as to form a complex by a non-covalent bond such as a hydrogen bond, a hydrophobic bond, an electrically static interaction or the like. The binding of the protein to MITF-M only at the portion of these molecules is enough to be referred to as "the binding" mentioned herein. For example, an amino acid which is not involved in the binding of the protein to MITF-M may be contained in the amino acid that constitutes the protein or MITF-M. The binding of protein selected from HLF, ELK4 and CLOCK to MITF-M can be detected by a method well known in the art, such as an immunoprecipitation method, a two-hybrid method, a Western blotting, a fluorescence resonance energy transfer method and the like, or by using these methods in combination. For example, the binding of protein selected from HLF, ELK4 and CLOCK to MITF-M can be determined by bringing the protein into reaction with MITF-M prepared as a GST fusion protein (GST-MITF-M), subsequently adsorbing the GST-MITF-M to glutathione Sepharose to recover it, and then detecting the protein existing in the recovered fraction (see Example 2). Detection of protein can be carried out by separating the protein using SDS-PAGE, and then detecting a band with a corresponding molecular weight of the protein.

The phrase "BCL2 gene product" refers to BCL2 that is expressed based on the information encoded by BCL2 gene through processes of transcription, translation and the like, and has a function. The function is exemplified by an anti-apoptotic activity. Preferably, the BCL2 gene product can be a gene product described above with an anti-apoptotic activity.

The phrase "melanoma cells" refers to malignant melanoma cells. The phrase "cell death of melanoma cells" refers to apoptosis of melanoma cells. Preferably, it refers to apoptosis of melanoma cells due to reduced or eliminated anti-apoptotic activity of BCL2 present in melanoma cells.

The phrase "sensitivity of melanoma to melanoma drugs" refers to reduction, disappearance, or no-growth of melanoma in response to melanoma drugs. The phrase "increase of such sensitivity" refers to increase of an extent of the reduction and/or a rate of the reduction or disappearance. As such melanoma drugs, any well known melanoma drugs can be used. The melanoma drugs can be single medication, or combination medications comprising more than two melanoma drugs. It is preferably exemplified by well known melanoma drugs to which sensitivity of melanoma increases with reduction of BCL2 amount in the melanoma. It is more preferably exemplified by such melanoma drugs as those to which sensitivity of melanoma increases with reduction of BCL2 amount in the melanoma where the reduction of BCL2 amount is given by inhibition of the binding of protein selected from HLF, ELK4 and CLOCK to MITF-M. Dacarbazine (DTIC) can be most preferably exemplified. Specifically, melanoma drugs given as single medication can be exemplified by nitrosoureas, nitrogen mustard drugs, triazenes, anthracycline drugs, vinca alikaroids, epipodophyllotoxins, taxanes, hormonal analogs, platinum drugs and the like. Examples of nirosoureas include carmustine (BCNU), lomustine (CCNU), semustine, fotemustine, (FTM), nimustine (ACNU) and the like. Examples of nitrogen mustard drugs include cyclophosphamide (CPA). Triazenes can be exemplified by dacarbazine (DTIC), temozolomide (TMZ) and the like. Examples of anthracycline drugs include doxorubicin (DXR) and bleomycin (BLM). Examples of vinca alikaroids include vincristine (VCR), vindesine (VDS), vinblastine (VLB) and the like. Examples of epipodophyllotoxins include etoposide. Examples of taxanes include paclitaxel (PTX) and docetaxel (TXT). Examples of hormonal analogs include anti-estrogen and tamoxifen (TAM). Examples of platinum drugs include cisplatin (CDDP) and carboplatin (CBDCA). The preferable example can be triazenes. The most preferable example can be DTIC. Melanoma drugs given as combination medication comprising two or more single medication include melanoma drugs comprising at least two or more single medication including DTIC. Preferable examples include a melanoma drug comprising VDS or VLB, CDDP and DTIC; a melanoma drug comprising BLM, VCR, CCNU and DTIC; a melanoma drug comprising DTIC, BCNU, CDDP and TAM; a melanoma drug comprising CDDP, ACNU, DTIC and TAM. Further, interleukin 2 (IL-2) or interferon α can be used in combination. The most preferable example can be a melanoma drug comprising DTIC, BCNU, CDDP and TAM. The most preferable example of melanoma drugs to which sensitivity of melanoma increases can be DTIC.

Inhibition of the binding of protein selected from HLF, ELK4 and CLOCK to MITF-M can be achieved, for example, by using an agent for inhibiting binding of protein selected from HLF, ELK4 and CLOCK to MITF-M. As used herein, a compound showing an inhibitory effect on a certain function (as examples described later, proteins, antibodies, compounds having a lower molecular weight, and the like, which have a competitive inhibitory effect, are listed) or a composition containing the compound is referred to as an inhibiting agent. The phrase "an agent for inhibiting binding of protein selected from HLF, ELK4 and CLOCK to MITF-M" refers to a compound having an effect of inhibiting binding of the protein to MITF-M, or a composition containing the compound.

Examples of a compound having an effect of inhibiting binding of protein selected from HLF, ELK4 and CLOCK to MITF-M include proteins, antibodies, compounds having a lower molecular weight, and the like, which have a competitive inhibitory effect. A compound can be preferably used that specifically inhibits binding of protein selected from HLF, ELK4 and CLOCK to MITF-M. A compound can be more preferably used that has a lower molecular weight and specifically inhibits the binding. The phrase "specifically inhibit binding of protein selected from HLF, ELK4 and CLOCK to MITF-M" denotes inhibiting that binding strongly, but does not inhibit or weakly inhibit the binding between the other proteins.

A compound having an effect of inhibiting binding of protein selected from HLF, ELK4 and CLOCK to MITF-M can be more specifically exemplified by a protein represented by an amino acid sequence of a site in the amino acid sequences of the protein and MITF-M where the protein binds to MITF-M. Such a protein can be obtained by designing proteins based on the amino acid sequence of protein selected from HLF, ELK4 and CLOCK and the amino acid sequence of MITF-M, synthesizing them by peptide synthesis methods well known in the art, and selecting a protein that inhibits binding of protein selected from HLF, ELK4 and CLOCK to MITF-M from them. Selection of such a protein can be performed by utilizing a method for identifying a compound that inhibits binding of protein selected from HLF, ELK4 and CLOCK to MITF-M as described later. A protein having an amino acid sequence derived from the thus specified protein, in which a mutation such as a deletion, substitution, addition or insertion of one to several amino acids has been introduced, is also included in the scope of the present invention. Among the proteins into which such a mutation has been introduced, a protein that inhibits the binding of protein selected from HLF, ELK4 and CLOCK to MITF-M is preferably used. A protein having the mutation may be a naturally existing protein or a protein in which a mutation has been introduced. Techniques for introducing a mutation such as a deletion, substitution, addition or insertion are known. For example, the Ulmer technique (Non-patent Reference 20) may be utilized. When introducing a mutation as described above, in view of avoiding a change in the fundamental properties (such as physical properties, function, and immunological activity) of the protein, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived. Furthermore, these usable proteins can be modified to the extent that no significant functional change is involved, for example, by modification of its constituent amino group or carboxyl group and the like, such as by an amidation and the like. Further, a protein is also included in the scope of the present invention that contains the specified protein as described above or the protein in which a mutation has been introduced, and has an effect of inhibiting the binding. Such a protein can be obtained by using a method of producing a protein described later.

A compound having an effect of inhibiting binding of protein selected from HLF, ELK4 and CLOCK to MITF-M can also be exemplified by an antibody that recognizes HLF, ELK4, CLOCK or MLTF-M and inhibits binding of the protein to MITF-M. Such an antibody can be obtained by known methods for preparing an antibody using each protein itself such as a protein selected from HLF, ELK4 and CLOCK, or MITF-M, or a fragment thereof, preferably a protein represented by an amino acid sequence of a site where protein selected from HLF, ELK4 and CLOCK binds to MITF-M, as an antigen.

Further aspect of the present invention relates to a method of identifying a compound that inhibits binding of protein selected from HLF, ELK4 and CLOCK to MITF-M. The identification method can be constructed by utilizing a known pharmaceutical screening system and using one of or combination of the followings: HLF, ELK4, CLOCK, or MITF-M; a gene encoding any one of them; a vector containing the gene; a transfectant prepared by transfecting the vector; or a cell expressing the gene.

The identification method described above can be, for example, an identification method comprising contacting a compound (hereinafter, referred to a test compound) with protein selected from HLF, ELK4 and CLOCK and/or with MITF-M under selected conditions that allow for binding of the protein to MITF-M and for interaction of the test compound with the protein and/or MITF-M, and then detecting the binding of the protein to MITF-M to determine whether the test compound inhibits the binding of the protein to MITF-M or not. Determination of whether the test compound inhibits the binding of the protein selected from HLF, ELK4 and CLOCK to MITF-M or not can be carried out by comparing a result of measurement of the binding when contacting the test compound to the binding when not contacting the test compound, and detecting a change thereof, such as reduction or elimination. The binding of the protein selected from HLF, ELK4 and CLOCK to MITF-M can be detected by a method well known in the art, such as an immunoprecipitation method, a pull down method, a Western blotting, and the like, or by using these methods in combination. In addition, determination of whether the test compound inhibits the binding of the protein selected from HLF, ELK4 and CLOCK to MITF-M or not can be carried out by employing a system that uses a signal and/or a marker generated by the binding, and then detecting presence, absence, or change of the signal and/or the marker. In the case that the signal and/or the marker shows a change, such as reduction or elimination, when contacting a test compound with the protein selected from HLF, ELK4 and CLOCK and/or with MITF-M, it can be determined that the test compound inhibits the binding of the protein to MITF-M.

As used herein, the term "signal" refers to a substance that can be detected itself directly based on its physical properties or chemical properties. A signal can be exemplified by a tag-peptide, a radioactive isotope, biotin, an enzyme, a fluorescent dye, and the like. Examples of a tag-peptide include GST-tag, MBP-tag (maltose binding protein-tag), FLAG-tag, His-tag, and the like. The term "marker" refers to a substance which itself can be not be directly detected based on its physical properties or chemical properties, but is capable of generating a signal like the above via chemical reaction and can be indirectly detected based on its physical properties, chemical properties, or biological properties as an index. A marker can be exemplified by a reporter gene, a labeling substance that generates luminescence in BRET (bioluminescence resonance energy transfer) or FRET (fluorescence energy transfer), and the like. Examples of a reporter gene include BCL2, luciferase, β-galactosidase, chloramphenicol acetyltransferase, and the like. Signals and/or markers are not limited to these examples, and can be any signals and markers generally used in methods of identifying a compound. A method of detecting these signals or markers is well known to those skilled in the art.

In the identification method described above, a test compound may be previously contacted with protein selected form HLF, ELK4 and CLOCK and/or with MITF-M, and then the binding reaction of the protein to MITF-M may be conducted. Alternatively, a test compound may be allowed to co-exist in the binding reaction to contact with the protein and/or MITF-M. The conditions that allow for binding of protein selected form HLF, ELK4 and CLOCK to MITF-M may be a condition in vitro or in vivo. For example, a cell in which protein selected form HLF, ELK4 and CLOCK is co-expressed with MITF-M may be used. Co-expression in a cell can be achieved by transfecting a cell using a suitable vector containing a polynucleotide encoding protein selected form HLF, ELK4 and CLOCK together with a suitable vector containing a polynucleotide encoding MITF-M by means of conventional genetic manipulation techniques.

Specifically, the identification method described above can be carried out, for example, by employing an in vitro binding assay system generally known in the art, which comprises immobilizing either of protein selected from HLF, ELK4 and CLOCK or MITF-M onto a solid-phase, conducting a binding reaction using the other one that is labeled with a signal, and measuring the labeled signal quantitatively. A compound that inhibits binding of protein selected from HLF, ELK4 and CLOCK to MITF-M can be identified by subjecting a compound to such a binding assay system to evaluate it.

The identification method described above can also be carried out, for example, by using GST-MITF-M, a GST-tag fusion protein expressed by means of genetic manipulation techniques. Specifically, it can be carried out by employing a binding assay system which comprises bringing HLF, ELK4 or CLOCK into reaction with GST-MITF-M, subsequently recovering GST-MITF-M by using glutathione Sepharose, and then detecting the protein being bound to recovered GST-MITF-M (see Example 2). A compound that inhibits binding of protein selected from HLF, ELK4 and CLOCK to MITF-M can be identified by subjecting a compound to such a binding assay system to evaluate it. Detection of the protein being bound to GST-MITF-M recovered by glutathione Sepharose can be carried out by separating the protein itself by SDS-PAGE and using antibodies raised against the protein being bound to GST-MITF-M. Alternatively, detection of the protein being bound to GST-MITF-M can be carried out quantitatively by using the protein described above that is labeled with a labeling substance such as enzymes, radio isotopes, fluorescent substances, biotin, tag-peptids, and the like, and measuring the labeling substance. A method of detecting these labeling substances is well known to those skilled in the art. For example, in the case of using the biotin-labeled protein described above, the protein can be directly detected by using horse radish peroxidase-conjugated streptavidin (see Example 2). In the case of using the tag-peptide-fused protein described above, the protein can be quantitatively measured by using anti-tag antibodies. In order to carry out quantitative measurement easily, it is preferable to label antibodies for use in measuring the protein described above, for example, with an enzyme such as horse radish peroxidase (HRP) or alkaline phosphatase (ALP), a radioactive isotope, a fluorescent substance, or biotin. Alternatively, a quantitative measurement can be carried out using a non-labeled primary antibody and a secondary antibody labeled with an enzyme such as HRP or ALP, a radio isotope, a fluorescent substance, biotin, or the like.

The identification method described above can be carried out by employing an assay system, which uses a cell in which a gene encoding a protein selected from HLF, ELK4 and CLOCK is co-expressed together with a gene encoding MITF-M, and detects binding reaction in the cell. A compound that inhibits in vivo binding of protein selected from HLF, ELK4 and CLOCK to MITF-M can be identified by subjecting a compound to the cell in such an assay system and then detecting the binding of the protein to MITF-M by using a method well known in the art, such as an immunoprecipitation method, a pull down method, or a Western blotting.

Alternatively, the identification method described above can also be carried out by employing a so-called reporter assay system. A reporter assay system can be exemplified by that using a cell transfected with plasmids which respectively contain a gene encoding a protein selected from HLF, ELK4 and CLOCK, a gene encoding MITF-M, and a reporter gene comprising a BCL2 gene promoter region linked to a luciferase gene (see Example 3). A test compound is contacted with such a cell, and then luciferase activity is measured. In the case that luciferase activity is reduced or eliminated when measuring the activity after contacting a test compound with such a cell, compared to when measuring the activity after not contacting a test compound, it can be determined that the test compound inhibits binding of the protein to MITF-M.

A well known two-hybrid method can be employed for carrying out the identification method described above. For example, transfection of a yeast, a eukaryotic cell, or the like are conducted using a plasmid for expressing a protein selected from HLF, ELK4 and CLOCK as a fusion protein with a DNA binding protein, a plasmid for expressing MITF-M as a fusion protein with transcription activating protein, and a plasmid containing a reporter gene linked to a suitable promoter gene. Then, an amount of expression of the reporter gene under co-existence of a test compound is compared to an amount of expression of the reporter gene in the absence of the test compound, which allow for identification of a compound that inhibits binding of protein selected from HLF, ELK4 and CLOCK to MITF-M. In the case that an amount of expression of the reporter gene under co-existence of a test compound is eliminated or reduced in comparison to an amount of expression of the reporter gene in the absence of the test compound, it can be determined that the test compound has an effect of inhibiting binding of the protein to MITF-M. A reporter gene can be any gene generally used in a reporter assay, and can be exemplified by, for example, genes encoding proteins having an enzyme activity, such as luciferase, β-galactosidase, chloramphenicol acetyltransferase, and the like. Detection of expression of a reporter gene can be carried out by measuring an activity of its gene product. For example, when using a reporter gene exemplified above, it can be carried out by measuring an enzyme activity of the gene product.

The identification method described above can also be carried out by employing a surface plasmon resonance sensor, such as BIACORE system or the like.

The identification method described above can also be carried out by employing a scintillation proximity assay (SPA), or a method that utilizing fluorescence resonance energy transfer (FRET).

Further aspect of the present invention relates to a method of identifying a compound that inhibits production of BCL2 gene product. The identification method comprises contacting a test compound with protein selected from HLF, ELK4 and CLOCK and/or with MITF-M under selected conditions that allow for binding of the protein to MITF-M and for interaction of the test compound with the protein and/or MITF-M, and then determining whether production of BCL2 gene product is inhibited or not.

Such an identification method can be specifically exemplified by an identification method that uses a cell transfected with plasmids which respectively contain a gene encoding a protein selected from HLF, ELK4 and CLOCK, a gene encoding MITF-M, and BCL2 gene. After subjecting a test compound to the cell, detection of BCL2 gene product is conducted. In the case that BCL2 gene product is decreased or eliminated, compared to when not subjecting a test compound, it can be determined that the test compound inhibits production of BCL2 gene product. Alternatively, such an identification method can also be carried out similarly by using a plasmid containing a BCL2 gene promoter region linked to a reporter gene instead of using a plasmid containing BCL2 gene, and then detecting a reporter gene product instead of BCL2 gene product (see Example 3).

Further aspect of the present invention relates to a method of identifying a compound that induces cell death of melanoma cells. The identification method comprises contacting a test compound with protein selected from HLF, ELK4 and CLOCK and/or with MITF-M under selected conditions that allow for binding of the protein to MTTF-M and for interaction of the test compound with the protein and/or MITF-M, and then determining whether cell death of melanoma cells is induced or not.

Such an identification method can be exemplified by, for example, an identification method that uses melanoma cells in which enhanced expression of BCL2 gene and MITF-M gene is found. After subjecting a test compound to such melanoma cells, detection of cell death or cell death signals is conducted. In the case that cell death is induced or enhanced, or in the case that cell death signals is increased, enhanced or generated, compared to when not subjecting a test compound, it can be determined that the test compound induces cell death of melanoma cells.

The phrase "cell death signals" refers to a morphological or a biological change unique to apoptotic cells. A morphological change unique to apoptotic cells is, for example, chromosome condensation in cell nucleus, DNA fragmentation, microvillus effacement, cytoplasmic condensation, apoptotic body formation, and the like. A biological change unique to apoptotic cells is, for example, DNA ladder, exposure of phosphatidyl serine on outer cell membrane resulting in change in cell membrane structure, loss in mitochondrial membrane potential, translocation of cytochrome c from mitochondoria to cytoplasm, and the like. These can be detected by well known methods in the art. For example, DNA fragmentation can be detected by ISEL method or TUNEL method, both of which are well known in the art. DNA ladder can be detected, for example, by extracting fragmented DNA from cells using well known methods, and then subjecting it to agarose electrophoresis and the like. Further, chromosome condensation in cell nucleus, microvillus effacement, cytoplasmic condensation, apoptotic body formation, and the like can be detected by observing cell morphology with an electron microscope. Induction or enhancement of cell death can be detected according to well known methods, by measuring reduction of viability of cultured melanoma cells. In such an identification method, melanoma cells transfected with a plasmid containing a gene encoding a protein selected from HLF, ELK4 and CLOCK can also be used.

In addition, the present invention allows for conducting a method of identifying a compound that increases sensitivity of melanoma to melanoma drugs. The identification method comprises contacting a test compound with protein selected from HLF, ELK4 and CLOCK and/or with MITF-M under selected conditions that allow for binding of the protein to MITF-M and for interaction of the test compound with the protein and/or M1TF-M, and then determining whether sensitivity of melanoma to melanoma drugs is increased or not.

Such an identification method can be specifically exemplified by, for example, an identification method comprising using melanoma cells in which enhanced expression of BCL2 gene and MITF-M gene is found, and contacting the melanoma cells to a test compound, subsequently subjecting the cells to a known melanoma drug, finally detecting cell death of melanoma to obtain a compound that increases sensitivity of melanoma to the melanoma drug. Dacarbazine (DTIC) is preferably used as a known melanoma drug. In the case that cell death of melanoma cells is induced, compared to when not contacting a test compound with melanoma cells, it can be determined that the test compound increases sensitivity of melanoma to the melanoma drug. In such an identification method, melanoma cells transfected with a plasmid containing a gene encoding a protein selected from HLF, ELK4 and CLOCK can also be used.

HLF, ELK4, CLOCK and MITF-M can be the following: products prepared from cells in which these are expressed by means of genetic manipulation techniques, products prepared from biological samples, products of cell-free synthesis systems, chemical synthesis products, or products further purified from them. Further, a cell in which at least one of HLF, ELK4, CLOCK and MITF-M is expressed by means of genetic manipulation techniques can also be used. These proteins can be those lacking a part of them, as long as it has no influence upon binding of protein selected from HLF, ELK4 and CLOCK to MITF-M and upon their function. Further, these can be ligated to a labeling substance at the N-terminus or the C-terminus under the same limitation as above. Examples of a labeling substance includes a different type of protein and the like, for example, GST, β-galactosidase, an Fc fragment of immunoglobulin such as IgG, tag-peptides such as His-tag, Myc-tag, HA-tag, FLAG-tag, or Xpress-tag, biotin, radio isotopes, and the like. These labeling substances can be linked thereto directly or indirectly via a linker peptide and the like, by means of, for example, genetic engineering techniques.

BCL2 can be the following: product prepared from cells in which BCL2 gene is expressed by means of genetic manipulation techniques, product prepared from biological samples, product of cell-free synthesis systems, chemical synthesis product, or product further purified from them. Further, a cell in which BCL2 gene is expressed by means of genetic manipulation techniques can also be used. BCL2 can be that lacking a part of it, as long as it has no influence upon the function such as interaction with MITF-M, an anti-apoptotic activity, or the like. Further, it can be ligated to a labeling substance at the N-terminus or the C-terminus under the same limitation as above. Examples of a labeling substance includes a different type ofprotein and the like, for example, GST, β-galactosidase, an Fc fragment of immunoglobulin such as IgG, tag-peptides such as His-tag, Myc-tag, HA-tag, FLAG-tag, or Xpress-tag, biotin, radio isotopes, and the like. These labeling substances can be linked thereto directly or indirectly via a linker peptide and the like, by means of, for example, genetic engineering techniques.

Specifically, proteins used in the present invention can be produced by purifying the proteins from animal-derived tissues or cells in which expression of the proteins is found, using well known protein purification methods. In such a method, animal-derived tissues or cells are homogenized first, followed by extraction of proteins with acids, organic solvents, or the like. Subsequently, a protein in interest is isolated and/or purified from the resultant extract by employing well known purification methods. Examples of isolation and/or purification methods include ammonium sulfate precipitation, ultrafiltration, gel chromatography, ion-exchange chromatography, affinity chromatography, high performance liquid chromatography, and dialysis. These methods may be used independently, or in suitable combinations. It is preferable to employ a method of specific adsorption using specific antibodies to a protein which are prepared using the protein or its fragments by means of well known antibody preparation method. For example, it is preferable that affinity chromatography that utilizes a column bound with specific antibodies can be used. Alternatively, the proteins can be produced according to conventional chemical synthesis methods well known in peptide chemistry. Further, it can be produced by using a commercially available amino acid synthesizer. The chemical synthesis method for protein may be a method described in publications (Non-patent References 21 and 22), but it is not limited thereto, and any well known methods can be used. Specifically, solid phase synthesis, solution phase synthesis, and the like, are known, and any of these methods can be used. These kinds of protein synthesis methods can be more specifically exemplified by a so-called stepwise elongation method that sequentially binds each amino acid, one at a time, to elongate a chain based on amino acid sequence information, and a fragment condensation method that previously synthesizes fragments consisting of several amino acids, and subsequently subjects the respective fragments to a coupling reaction. The present proteins can be synthesized by either of these methods. A condensation method used for the aforementioned protein synthesis methods can also be carried out according to conventional methods. Examples of condensation methods include an azide method, mixed anhydride method, DCC method, active ester method, oxidation-reduction method, DPPA (diphenylphosphoryl azide) method, DCC + additive (1-hydroxybenzotriazole, N-hydroxysuccinamide, N-hydroxy-5-norbomane-2,3-dicarboxyimide, and the like) method, and Woodward's method. The present protein obtained by chemical synthesis can be suitably purified in accordance with various kinds of conventional purification methods as described above. Alternatively, the present proteins can be prepared, for example, by standard gene manipulation techniques (refer to Non-Patent References 20, 23 and 24) based on the nucleotide sequence information of genes encoding the present proteins. For example, a protein in interest can be produced by preparing a recombinant vector that contains a gene encoding the protein and is capable of expressing the proteins in host cells, and preparing a transfectant by transfecting the recombinant vector thereto, and then subjecting the transfectant to induction of the gene expression, subsequently collecting the protein from the transfectant. When a protein encoded by the gene is expressed in a transformant prepared by transfecting a recombinant vector containing the gene, or on its cell membrane, the proteins may be extracted from the disrupted transformant. Further, when the protein is secreted outside a transformant prepared by transfecting a recombinant vector containing the gene, the cultured medium can be used as is, or the cultured medium, after removing the transformant by centrifugation or the like, can be used. As desired, the protein can be purified from a cultured medium of a transformant or from the transformant, by the purification methods described above. Alternatively, the protein can be prepared by using a well known cell-free protein expression system, using a recombinant vector into which a gene encoding the protein is introduced (Non-patent Reference 25).

The genes encoding proteins used in the present invention can be acquired by preparing a cDNA library in accordance with an ordinary method, from a suitable source in which expression of the genes was found, and then selecting a desired clone from the cDNA library. As a cDNA source, various kinds of cells and tissues in which expression of the proteins was found, or cultured cells derived from these cells and tissues, can be used. Preferably, for example, melanoma tissue, liver tissue, brain tissue, cultured cells derived from these tissues, or the like, can be used. Most preferably, for example, a source for MITF-M cDNA can be melanoma tissue or melanoma cells; a source for BCL2 cDNA can be melanoma tissue or melanoma cells; a source for HLF cDNA can be liver tissue, melanoma tissue, or cells derived from these tissues; a source for ELK4 cDNA can be melanoma tissue or melanoma cells; and a source for CLOCK cDNA can be brain tissue or brain-derived cells. CLOCK cDNA can be also prepared from a cDNA library constructed from commercially available polyA⁺ RNA derived from human brain tissue, fetal brain tissue and cerebral hippocampus tissue. Isolation of total RNA from these sources, isolation and purification of mRNA, acquisition of cDNA, the cloning thereof, and the like, in preparing cDNA library can each be performed in accordance with an ordinary method. A method of selecting a desired clone from a cDNA library is not particularly limited, and any methods generally used can be employed. For example, selection of a desired clone can be performed by using a probe or primer capable of selectively hybridizing to a gene encoding the protein. Specifically, a plaque hybridization method, colony hybridization method, or the like, which uses a probe capable of selectively hybridizing to the gene, or a combination of these methods, can be employed. As a probe or a primer, a polynucleotide chemically synthesized based on the sequence information of the gene, and the like, can generally be used. A recombinant vector containing a gene encoding the protein can be constructed by inserting the gene prepared by the method described above into a suitable vector DNA. The vector DNA is not particularly limited as long as it can be replicated after being integrated into a host or host genome and can express the gene. The vector DNA can be suitably selected in accordance with the kind of host and purpose of use. The vector DNA may be vector DNA obtained by extracting natural DNA, or may be vector DNA lacking a part of DNA other than a segment necessary for replication. Typical vector DNAs include, for example, a vector DNA derived from a plasmid, a bacteriophage or a virus. A plasmid DNA can be exemplified by a plasmid derived from *Escherichia coli,* a plasmid derived from *Bacillus subtilis,* or a plasmid derived from yeast. A bacteriophage DNA can be exemplified by a λ phage. Vector DNA derived from a virus can be exemplified by a vector derived from an animal virus, such as a retrovirus, vaccinia virus, adenovirus, papovavirus, SV 40, fowlpox virus, and pseudorabies virus, or a vector derived from an insect virus such as baculovirus. Further, vector DNA derived from a transposon, an insertion element, a yeast chromosome element, or the like, may be used. Alternatively, a vector DNA prepared by combining two or more of these, for example, a vector DNA (cosmid, phagemid or the like) prepared by combining genetic elements of a plasmid and a bacteriophage, may be used. It is necessary for a gene to be incorporated into vector DNA in such a way as to allow the function of the gene to appear. The vector DNA contains at least the gene and a promoter, as construction elements. In addition to these elements, as desired, a genetic sequence that encodes information relating to replication and control, may be incorporated in combination into the vector DNA, by using a well known method. Such a genetic sequence can be exemplified by a ribosome binding sequence, terminator, signal sequence, cis element such as an enhancer, splicing signal, and a selective marker such as dihydrofolate reductase gene, ampicillin-resistant gene and neomycin-resistant gene. The vector DNA may contain one or more kinds of genetic sequences selected from the aforementioned members. As a method of incorporating the gene into a vector DNA, any known method can be employed. For example, a method may be used which comprises cleaving the gene at specific sites, by treating it with suitable restriction enzymes, and then mixing it with a similarly treated vector DNA, for ligation using a ligase. Alternatively, a desired recombinant vector may be prepared by using a method that comprises ligating the gene with a suitable linker, and then inserting it into the multi-cloning site of a vector, suitable for the desired purpose. A transformant, prepared by transfecting a host with a vector DNA incorporating the gene, is useful for producing a protein encoded by the gene. Any suitable prokaryotes and eukaryotes can be employed as a host. Examples of suitable prokaryotes include bacteria belonging to the *Escherichia* genus, such as, *Escherichia coli,* bacteria belonging to the *Bacillus* genus, such as, *Bacillus subtilis,* bacteria belonging to the *Pseudomonas* genus, such as, *Pseudomonas putida,* and bacteria belonging to the *Rhizobium* genus, such as, *Rhizobium meliloti.* Examples of suitable eukaryotes include yeasts, insect cells, and mammalian cells. Yeasts can be exemplified by *Saccharomyces cerevisiae* and *Schizosaccharomyces pombe.* Insect cells can be exemplified by Sf9 cells and S12 cells. Mammalian cells can be exemplified by monkey kidney-derived cells, such as COS cells, Vero cells, Chinese hamster ovary cells (CHO cell), mouse L cells, rat GH3 cells, human FL cells, human 293EBNA cells, and the like. It is preferable to use mammalian cells. Transfection of a host cell with vector DNA can be carried out by employing well known methods, for example, in accordance with a standard method described in publications (Non-Patent Reference 23). When gene stability is a consideration, it is preferable to use a method that integrates the gene onto a chromosome. Meanwhile, it is convenient to use an autonomous replication system that utilizes an extranuclear gene. Specifically, calcium phosphate transfection, DEAE-dextran mediated transfection, microinjection, cationic lipid-mediated transfection, electroporation, scrape loading, ballistic introduction, infection, and the like, may be mentioned.

Examples of a test compound include a compound derived from a chemical library or natural products, as well as a compound obtained by drug design based on the primary structure or tertiary structure of HLF, ELK4, CLOCK or MITF-M. Alternatively, a compound obtained by drug design based on the structure of a protein represented by an amino acid sequence of a binding site of protein selected from HLF, ELK4 and CLOCK to MITF-M is also suitable as a test compound.

Compounds obtained by the identification method described above have an effect of inhibiting binding of protein selected from HLF, ELK4 and CLOCK to MITF-M. That is to say, compounds obtained by the identification method described above can be used as inhibitors for binding of protein selected from HLF, ELK4 and CLOCK to MITF-M. Such binding inhibitors can be utilized for agents for inhibiting production of BCL2 gene product, agents for inducing cell death of melanoma cells, and agents for increasing sensitivity of melanoma to melanoma drugs. Further, such binding inhibitors can be prepared as medicaments by taking into consideration the balance between usefulness and toxicity. In preparation of the pharmaceutical compositions, these binding inhibitors can be used alone or in combination. Further, such binding inhibitors can be used for conducting a method of inhibiting production of BCL2 gene product, a method of inducing cell death of melanoma cells, and a method of increasing sensitivity of melanoma to melanoma drugs.

The agent for treating and/or preventing diseases, which is provided according to the present invention, can be an agent for treating and/or preventing diseases which comprises at least any one member selected from the compounds, the inhibiting agents, the agents for inducing cell death and the agents for treating melanoma described above. The method of treating and/or preventing diseases according to the present invention can be a method of treating and/or preventing diseases which comprises using at least any one member selected from the compounds, the inhibiting agents, the agents for inducing cell death and the agents for treating melanoma described above.

The agent for treating and/or preventing diseases, which is provided according to the present invention, can be prepared as a medicament containing an effective amount of at least any one member selected from the compounds, the inhibiting agents, the agents for inducing cell death and the agents for treating melanoma described above as an effective ingredient. In general, it is preferable to prepare a pharmaceutical composition using one or more kinds of pharmaceutically acceptable carriers.

An amount of the effective ingredient contained in the pharmaceutical composition according to the present invention can be suitably selected from a wide range. In general, a suitable amount may fall within a range of approximately 0.00001 to 70 wt%, preferably approximately 0.0001 to 5 wt%.

A pharmaceutical carrier may be that which can be generally used in accordance with the form of use of the pharmaceutical composition, such as, a filler, an extender, a binder, a wetting agent, a disintegrator, a lubricant, a diluent and/or an excipients. These can be suitably selected and used in accordance with the form of use of the pharmaceutical composition.

The pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be suitably selected, and used in accordance with the form of use of a pharmaceutical composition of the present invention.

As desired, various ingredients used in conventional protein preparations can be suitably used herein, such as, a stabilizer, a bacteriocide, a buffer agent, an isotonizing agent, a chelating agent, a surfactant, a pH adjuster and the like.

As a stabilizer, the following may be used: human serum albumin, common L-amino acids, sugars, cellulose derivatives and the like. These can be used independently or in combination with a surfactant, and the like. Use of these in such a combination may give increased stability to an effective ingredient. An L-amino acid is not particularly limited, and may be any one of glycine, cysteine, glutamic acid, and the like. A sugar is not particularly limited, and may be any one of the monosaccharides (such as glucose, mannose, galactose, and fiuctose), sugar alcohols (such as mannitol, inositol, and xylitol), disaccharides (such as sucrose, maltose, and lactose), polysaccharides (dextran, hydroxypropylstarch, chondroitin sulfate, and hyaluronic acid), derivatives thereof, and so on. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like.

A surfactant is not particularly limited, and can be both an ionic surfactant and/or a nonionic surfactant. As a surfactant, the following may be used: polyoxyethyleneglycol sorbitan alkyl ester base; polyoxyethylene alkyl ether base; sorbitan monoacyl ester base; a fatty acid glyceride base; or the like.

As a buffer agent, the following may be used: boric acid; phosphoric acid; acetic acid; citric acid; ∈-aminocaproic acid; glutamic acid; and/or a salt thereof, for example, an alkali metal salt and/or an alkaline earth metal salt, such as a sodium salt, a potassium salt, a calcium salt and a magnesium salt.

As an isotonizing agent, the following may be used: sodium chloride; potassium chloride; sugars; glycerin; or the like.

As a chelating agent, sodium edetate and citric acid may be used.

The medicaments and the pharmaceutical compositions, which are provided according to the present invention, can be used as solution preparations. Alternatively, they can be freeze-dried, so as to be preservable. They can be used by dissolving them in water, a buffered solution containing saline, and the like, and then adjusting them to a suitable concentration, at the time of use.

The medicaments and the pharmaceutical compositions, which are provided according to the present invention, can be used as an agent for treating and/or preventing diseases accompanied by enhanced production of BCL2 gene product. Further, they can be used in a method of treating and/or preventing diseases accompanied by production of BCL2 gene product. Preferably, they can be suitably applied for diseases accompanied by enhanced production of BCL2 gene product due to binding of protein selected from HLF, ELK4 and CLOCK to MITF-M. Melanoma can be specifically exemplified as such a disease.

BCL2 has been found to be highly expressed in various cancers as described above. Therefore, anti-cancer drugs based on a pharmaceutical mechanism of inhibiting expression and/or activity of BCL2 allow for improving not only melanoma but various cancers. Meanwhile, BCL2 has been found to be expressed in various normal tissues. Therefore, administration of such an anti-cancer drug for the purpose of improving or treating melanoma may bring side effects to the normal tissues other than melanocytes. However, the agent for treating and/or preventing melanoma, which is provided according to the present invention, inhibits transcriptional activation of BCL2 gene by MITF-M via inhibiting binding of a protein selected from HLF, ELK4 and CLOCK to MITF-M, which is a novel pharmacological mechanism. In addition, MITF-M is expressed specifically in melanoma and melanocytes. Therefore, the agent for treating and/or preventing melanoma, which is provided according to the present invention, has a reduced side effect compared to conventional melanoma drugs.

Suitable dosage ranges of the medicament and the pharmaceutical composition are not particularly limited, and can be determined in accordance with the following: effectiveness of the ingredients contained therein; the administration form; the route of administration; the type of disease; the characteristics of the subject (e.g., body weight, age, symptomatic conditions, and whether a subj ect is taking other pharmaceutical agents); and the judgment of a physician in charge. In general, a suitable dosage may fall, for example, within a range of about 0.01 µg to 100 mg, per 1 kg of the body weight of the subject, and preferably within a range of about 0.1 µg to 1 mg, per 1 kg of body weight. However, the dosage may be altered using conventional experiments for optimization of a dosage that are well known in the art. The aforementioned dosage can be divided for administration once to several times a day. Alternatively, periodic administration once every few days or few weeks can be employed.

When administering the medicament or the pharmaceutical composition according to the present invention, the medicament or the pharmaceutical composition may be used alone, or may be used together with other compounds or medicaments useful for preventing and/or treating the target disease. It is preferable to use in combination. For example, when using the medicament or the pharmaceutical composition in methods of treating and/or preventing melanoma, it can be used together with drugs and/or preventives for melanoma that are different from the medicament or the pharmaceutical composition. The medicament or the pharmaceutical composition can inhibit production of BCL2 gene product and enhance apoptosis of melanoma, which results in increase of sensitivity of melanoma to melanoma drugs that are different from the medicament or the pharmaceutical composition. That is to say, the medicament or the pharmaceutical composition can be utilized as an auxiliary agent used together with melanoma drugs that are different from the medicament or the pharmaceutical composition. Melanoma drugs used together with the medicament or the pharmaceutical composition are not particularly limited, and any well known drugs can be used. For example, the well known melanoma drugs described above can be used. It is preferably exemplified by melanoma drugs to which sensitivity of melanoma increases when inhibiting production of BCL2 gene product with the medicament or the pharmaceutical composition, compared to when not using the medicament or the pharmaceutical composition. Such a melanoma drug can be selected by performing a method of identifying a compound that increases sensitivity of melanoma to melanoma drugs, as described above. It has been reported that inhibition of expression of BCL2 gene product resulted in increased sensitivity of melanoma to dacarbazine (DTIC) (Non-patent Reference 4). Therefore, it is preferable to use in combination with dacarbazine. The combination medications described above can give a significant effect in melanoma therapy.

In terms of a route of administration, it may be either systemic administration or local administration. The route of administration that is appropriate for a particular disease, symptomatic condition, or other factors, should be selected. For example, parenteral administration including normal intravenous injection, intra-arterial administration, subcutaneous administration, intracutaneous administration, intramuscular administration, and so on can be employed. Oral administration can be also employed. Further, transmucosal administration or dermal administration can be employed. In the case of use for cancer disease, it may be preferable to employ a direct administration into the tumor by injection, and the like.

In terms of an administration form, various forms can be selected in accordance with a treatment purpose. For example, a solid formulation may be employed such as a tablet, pill, powder, powdered drug, fine granule, granule, or a capsule. Alternatively, a liquid formulation can be employed such as an aqueous formulation, ethanol formulation, suspension, fat emulsion, liposome formulation, clathrate such as cyclodextrin, syrup, or an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation, and the like, which can be respectively blended, formed and prepared according to conventional methods.

Further aspect of the present invention relates to a reagent kit. The reagent kit comprises at least any one member selected from a protein selected from a group consisting of HLF, ELK4 and CLOCK, a polynucleotide encoding the protein, a recombinant vector comprising the polynucleotide, and a transformant comprising the recombinant vector; and at least any one member selected from MITF-M, a polynucleotide encoding MITF-M, a recombinant vector comprising the polynucleotide and a transformant comprising the recombinant vector. The reagent kit can be used, for example, in the identification method for the present invention. The protein can be prepared by the aforementioned production method. The polynucleotide, the recombinant vector and the transformant can be prepared by using genetic manipulation techniques described above. The reagent kit may contain a substance necessary for carrying out the identification methods described above, such as a signal and/or a marker, a buffer solution, salts and the like. The reagent kit may also contain a substance, such as stabilizers and/or antiseptic agents. At the time of preparation, methods for preparation may be introduced in accordance with the respective substances to be used.

Hereinafter, the present invention may be explained more specifically with the following examples.

### Example 1

### (In-silico Search for Proteins Having a Function to Interact with MITF-M)

The prediction of proteins that have a function to interact with MITF-M was conducted according to the method described in the Patent Document 1 as follows: i) decomposing an amino acid sequence of MTTF-M into a predetermined length of oligopeptide, ii) searching a database for proteins having an amino acid sequence of the each oligopeptide or a homologous amino acid sequence to the amino acid sequence, iii) conducting a local alignment between the resultant proteins and MITF-M, and iv) predicting proteins having a high local alignment score to be those capable of interacting with MITF-M.

As a result of analysis, HLF, ELK4 and CLOCK were identified as proteins being predicted to have a function of interacting with MITF-M. HLF has an oligopeptide (LENPLKL (SEQ ID NO: 22)) in its amino acid sequence, that is homologous to an oligopeptide (LENPTKY (SEQ ID NO: 21)) comprising amino acid residues derived from MITF-M (Fig. 1-A). ELK4 has an oligopeptide (PGAKTSSR (SEQ ID NO: 24)) in its amino acid sequence, that is homologous to an oligopeptide (PGASKTSSR (SEQ ID NO: 23)) comprising amino acid residues derived from MITF-M (Fig. 1-B). CLOCK has oligopeptides (IKELGS (SEQ ID NO: 27) and SSRKSS (SEQ ID NO: 28)) in its amino acid sequence, that are homologous to oligopeptides (IKELGT (SEQ ID NO: 25) and SSRRSS (SEQ ID NO: 26)) comprising amino acid residues derived from MITF-M (Fig. 1-C).

### Example 2

### (Binding Analysis of MITF-M to HLF, ELK4 or CLOCK)

A study was conducted on whether MITF-M binds to HLF, ELK4 or CLOCK, or not, using a method described below.

In the present example, GST fusion protein, GST-MITF-M, was prepared and used as MITF-M.

HLF, ELK4 and CLOCK were respectively synthesized in vitro using transcend Biotin-Lysyl-tRNA (Promega) and using TNT quick coupled transcription/translation system (Promega). At first, plasmids containing genes encoding each protein were prepared. As a plasmid, an expression plasmid having T7 promoter was used.

MITF-M gene was prepared by amplification by polymerase chain reaction (PCR) using Human XG Malignant melanoma (A375) QUICK-Clone cDNA (CLONTECH) as a template. The forward primer and the reverse primer used in PCR were oligonucleotides respectively represented by nucleotide sequences set forth in SEQ ID NOs: 9 and 10. An amplified product was cloned into pCR4 Blunt-TOPO (Invitrogen). A resultant clone has a nucleotide sequence completely same as a sequence disclosed in GenBank (accession number: NM_000248).

ELK4 gene was prepared by amplification by PCR using Human XG Malignant melanoma (A375) QUICK-Clone cDNA (CLONTECH) as a template. The forward primer and the reverse primer used in PCR were oligonucleotides respectively represented by nucleotide sequences set forth in SEQ ID NOs: 11 and 12. An amplified product was cloned into pCR BluntII-TOPO (Invitrogen). A resultant clone has a nucleotide sequence completely same as a sequence disclosed in GenBank (accession number: NM_021795.2).

HLF gene was prepared by amplification by PCR using Human Liver QUICK-Clone cDNA (CLONTECH) as a template. The forward primer and the reverse primer used in PCR were oligonucleotides respectively represented by nucleotide sequences set forth in SEQ ID NOs: 13 and 14. An amplified product was cloned into pCR4 Blunt-TOPO (Invitrogen). A resultant clone has a nucleotide sequence completely same as a sequence disclosed in GenBank (accession number: NM_002126.3).

CLOCK gene was purchased from KAZUSA DNA Research Institute and used. The gene has been disclosed as KIAA 0334 in a Database of Human Unidentified Gene-Encoded Large Proteins (HUGE) analyzed by the Research Institute. In addition, the nucleotide sequence of the gene was registered and has been disclosed (accession number: AB002332) in GenBank.

MITF cDNA was inserted into pGEX-4T-1 (Amersham Biosciences) and transfected into E coli. BL21 (DE3) strain (Novagen). As a negative control transfection with pGEX-4T-1 in a similar manner was used. The transfected BL21 strain was incubated at 37 °C, and then subj ected to induction of protein expression with 1 mM isopropyl 1-thio-β-D-galactoside (TPTG) by incubating at 26 °C for 3 hours. After disrupting the strain, GST-MITF-M and GST was purified by using glutathione Sepharose 4B (Amersham Biosciences).

HLF gene, ELK4 gene and CLOCK gene were inserted into pcDNA3.1/His (Invitrogen), pcDNA3.1/V5·His (Invitrogen) and pcDNA3.1/Myc·His (Invitrogen), respectively. TNT solution having a composition shown in Table 1 was prepared for each resultant plasmid.

**Table 1**

| TNT reaction solution | |
|---|---|
| TNT Quick Master (for T7) | 40 µl |
| 1mM methionine | 1 µl |
| plasmid (1.5µg equivalent) | 1.5 µl |
| Transcend Biotin-Lysyl-tRNA | 2 µl |
| distilled water | 5.5 µl |
| total | 50 µl |

GST-MITF-M was mixed with each of the TNT reaction solutions and binding buffer (40mM HEPES (pH7.5), 50mM KCl, 5mM MgCl₂, 0.2mM ethylenediaminetetraacetate, 1mM DTT (dithiothreitol) and 0.5 % Nonidet P-40) in a manner as shown in Table 2, and kept on ice for 1 hour.

**Table 2**

| | |
|---|---|
| GST-MITF-M (30 ng/µl) | 33 µl |
| TNT solution | 20 µl |
| binding buffer | 447 µl |
| total | 500 µl |

Purified GST used as a negative control was mixed with each of the TNT reaction solutions and the binding buffer in a manner as shown in Table 3, and kept on ice for 1 hour.

**Table 3**

| | |
|---|---|
| GST (0.5 µg/µl) | 2 µl |
| TNT solution | 20 µl |
| binding buffer | 478 µl |
| total | 500 µl |

After that, 500 µl of the resultant mixture was added with 20 µl of glutathione Sepharose 4B (Amersham biosciences) followed by mixing overnight at 4 °C under rotation. The glutathione Sepharose 4B was previously blocked with 0.1 % bovine serum albumin (BSA) in the binding buffer and then equilibrated with the binding buffer before use.

The resin was subjected to centrifugation at 10,000 rpm at 4 °C for 1 minute to recover it, and washed with 0.5 ml of the binding buffer for 4 times. Then, 20 µl of 2 × SDS-PAGE sample buffer containing 10 % β-mercaptoethanol was added thereto and boiled for 3 minutes. After that, the resin was precipitated by centrifugation, and resultant supernatant was loaded onto 5-20 % polyacrylamide gel to separate proteins contained in the supernatant. The proteins were transferred from polyacrylamide gel to PVDF membrane to detect co-precipitated protein by using horseradish peroxidase conjugated streptavidin (streptavidin-HRP) and ECL Western Blotting Detection System (Amersham Biosciences). Meanwhile, in order to determine the position of HLF, ELK4 or CLOCK separated in polyacrylamide gel, and to confirm the expression amounts of these three kinds of proteins to be enough for carrying out a binding assay, each of the TNT reaction solutions was loaded onto polyacrylamide gel to detect each expressed protein in the same manner as described above.

The results are shown in Figure 2. Bands indicating proteins that bind to GST-MITF-M were detected at positions corresponding to molecular weights of proteins of HLF, ELK4 and CLOCK. On the other hand, such bands were not detected in the case of reaction with GST. These results revealed that MITF-M bound to HLF, ELK4, or CLOCK.

### Example 3

### (Analysis of Effect of MTTF-M Binding to HLF, ELK4, or CLOCK on Transcription Activating Activity of MITF-M)

Effect of MITF-M binding to HLF, ELK4, or CLOCK on transcription activating activity of MITF-M was analyzed by means of a reporter assay using BCL2 gene promoter.

### <Construction of A Reporter Assay System>

A MITF-M gene expression plasmid used for constructing a reporter assay system was prepared by inserting MITF-M gene into pCI mammalian expression vector (Promega) in accordance with a conventional method. A reporter gene expression plasmid was prepared by inserting a BCL2 gene promoter region into a firefly luciferase reporter vector, pGL3-Basic vector (Promega) in accordance with a conventional method. A renilla luciferase reporter vector, phRL-null vector (Promega) was used as an internal control.

The three kinds of plasmids thus prepared were mixed in various proportions. The total amount of DNA was adjusted to 3.6 µg/well with a vector, pCI. The plasmid mixture was transfected into HEK293 cells. HEK293 cells were plated at 4 ×10⁵ cells /well in a 6 well plate and cultured at 37 °C for 5 hours before use. Transfection was carried out in such a way that the above plasmid mixture was added to a transfection solution consisting of 10 µl of FuGENE 6 (Roche) and 90 µl of serum-free D-MEM, and kept at room temperature for 20 minutes, subsequently dropped onto the above cells, followed by cultivating the cells at 37 °C for 42-48 hours. After cultivation, the cells were recovered and subjected to measurement of luciferase activity using Dual-Luciferase reporter assay system (Promega). Luciferase activity was calculated as firefly luciferase activity (F)/renilla luciferase activity (R).

As a result, transcriptional activity of the BCL2 gene promoter increased depending on the amount of MITF-M gene plasmid used. The luciferase activity in the cells transfected with 2.5 µg/well of the plasmid increased approximately 2.5 times compared to that in the cells not transfected with the plasmid. Further, the luciferase activity increased depending on the amount of reporter gene expression plasmid used. However, the amount of reporter gene expression plasmid used did not affect to the transcription activating activity of MITF-M gene expression plasmid.

Based on these results, it was determined that the amounts of MITF-M gene expression plasmid, the reporter gene expression plasmid and the internal control vector to be used in constructing a reporter assay system were 2.5 µg/well, 0.5 µg/well and 1 ng/well, respectively.

### <Study with A Reporter Assay>

Plasmids were prepared that contain genes encoding respective proteins of HLF, ELK4 and CLOCK. HLF gene was inserted into a pCI mammalian cell expression vector (Promega) so as to give a carboxy-terminal FLAG-tagged HLF protein. ELK4 gene was inserted into a pCI mammalian cell expression vector (Promega) so as to give a carboxy-terminal FLAG-tagged ELK4 protein. A CLOCK gene expression plasmid was prepared by inserting CLOCK gene into a pCI mammalian cell expression vector (Promega).

HEK293 cells were plated at 4×10⁵ cells /well in a 6 well plate and cultured at 37°C for 5 hours before use. Any one of the expression plasmids of HLF gene, ELK4 gene and CLOCK gene was mixed with the MITF-M gene expression plasmid, the reporter gene expression plasmid and the internal control vector so that the amount thereof were 2.5µg/well, 2.5µg/well, 0.5µg/well and 1ng/well, respectively, and then used for transfecting the cells. Meanwhile, a plasmid mixture containing any one of the expression plasmids of HLF gene, ELK4 gene and CLOCK gene, but not containing the MITF-M gene expression plasmid was prepared in a similar manner, and used for transfecting the cells that were used as a control. Further, a plasmid mixture not containing any of the expression plasmids of HLF gene, ELK4 gene and CLOCK gene, and a plasmid mixture not containing any of the plasmids of MITF-M gene, HLF gene, ELK4 gene and CLOCK gene were prepared in a similar manner, and individually used for transfecting cells. The cells were individually used as controls. The total amount of DNA was adjusted to 5.5µg/well using vector pCI.

Transfection of the cells with the plasmids was carried out in such a way that the above plasmid mixture was added to a transfection solution consisting of 16.5 µl of FuGENE 6 (Roche) and 83.5 µl of serum-free D-MEM, and kept at room temperature for 20 minutes, subsequently dropped onto the above cells, followed by cultivating the cells at 37 °C for 42-48 hours. After cultivation, the cells were recovered and subjected to measurement of luciferase activity using Dual-Luciferase reporter assay system (Promega). Luciferase activity was calculated as firefly luciferase activity (F)/renilla luciferase activity (R).

Results were shown in Figures 3-A, 3-B and 3-C. In the cells transfected with the MITF-M gene expression plasmid together with the ELK4 gene expression plasmid, luciferase activity increased approximately 1.4 fold than that in cells transfected only with the MITF-M gene expression plasmid (black column in Figure 3-A). Further, in the cells transfected with the MITF-M gene expression plasmid together with the HLF gene expression plasmid, luciferase activity increased approximately 1.8 fold than that in cells transfected only with the MITF-M gene expression plasmid (black column in Figure 3-B). In the cells transfected with the MITF-M gene expression plasmid together with the CLOCK gene expression plasmid, luciferase activity increased approximately 1.5 fold than that in cells transfected only with the MITF-M gene expression plasmid (black column in Figure 3-C). These increases were found to be statistically significant (n=3, p<0.05 or p<0.01 in t-test). On the other hand, luciferase activity in the cells that were not transfected with the MITF-M gene expression plasmid was not affected by transfection of ELK4 gene, HLF gene or CLOCK gene (each white column in Figures 3-A, 3-B, and 3-C).

Thus, the reporter assay using BCL2 gene promoter revealed that transcriptional activity of the BCL2 gene promoter was enhanced by expression of HLF, ELK4 or CLOCK together with MITF-M, compared to expression of MITF-M only. As is evident from the results of Example 2, all of HLF, ELK4 and CLOCK bind to MITF-M. Meanwhile, no enhanced transcriptional activity of the BCL2 gene promoter was observed by expressing HLF, ELK3 or CLOCK only.

These results suggest that the binding of HLF, ELK4 or CLOCK to MITF-M leads to enhanced transcriptional activity of BCL2 gene promoter. In other words, it was found that the binding of HLF, ELK4 or CLOCK to MITF-M leads to enhanced production of gene product of BCL2 gene to which MITF-M affects as a transcription factor.

### [Exmaple 4]

Expression of endogenous CLOCK, ELK4 and HLF in melanoma cells was studied. Specifically, PCR was carried out using Human XG malignant melanoma A375 and GI-105 Quick-clone cDNA (CLONTECH) as a template, primers described below, and KOD-Plus (TOYOBO) as a polymerase. PCR solution and PCR condition are shown in Tables 4 and 5, respectively. After that, expression of mRNA was detected by 1% agarose gel electrophoresis. In addition, a gene sequence was determined by analyzing nucleotide sequences of DNA amplified by PCR.

<primer>
Forward primer for amplifying CLOCK gene: SEQ ID NO: 15
Reverse primer for amplifying CLOCK gene: SEQ ID NO: 16
Forward primer for amplifying ELK4 gene: SEQ ID NO: 17
Reverse primer for amplifying ELK4 gene: SEQ ID NO: 18
Forward primer for amplifying HLF gene: SEQ ID NO: 19
Reverse primer for amplifying HLF gene: SEQ ID NO: 20

**Table 4**

| <PCR solution> | |
|---|---|
| 10 x buffer (for KOD plus) | 2.5 µl |
| 2 mM dNTP | 2.5 µl |
| 25 mM MgSO₄ | 1.2 µl |
| 5 µM forward primer | 1.5 µl |
| 5 µM reverse primer | 1.5 µl |
| template DNA | 0.5 µl |
| distilled water | 14.8 µl |
| KOD plus | 0.5 µl |
| total | 25 µl |

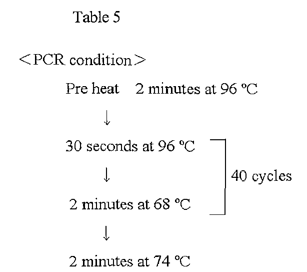

### <Results>

HLF gene, ELK 4 gene and CLOCK gene were detected in cDNAs derived from melanoma cells, A 375 and GI-105 (Figure 4). In addition, the nucleotide sequences of HLF gene, ELK 4 gene and CLOCK gene that were amplified by PCR were found to be completely same to the sequences disclosed in GenBank (accession numbers: NM_002126.3, NM_021795.2 and AB002332, respectively). These results revealed that HLF gene, ELK 4 gene and CLOCK gene were expressed in melanoma cells.

### INDUSTRIAL APPLICABILITY

The present invention can be utilized for treatment and/or prevention of diseases accompanied by increased BCL2 gene product, such as melanoma, and thus is extremely useful in pharmaceutical field.

GENERAL DESCRIPTION OF THE SEQUENCES
SEQ ID NO: 1: A gene encoding MITF-M (SEQ ID NO: 2).
SEQ ID NO: 3: A gene encoding HLF (SEQ ID NO: 4).
SEQ ID NO: 5: A gene encoding ELK4 (SEQ ID NO: 6).
SEQ ID NO: 7: A gene encoding CLOCK (SEQ ID NO: 8).
SEQ ID NO: 9: Designed oligonucleotide for use as a primer to amplify MITF-M gene.
SEQ ID NO: 10: Designed oligonucleotide for use as a primer to amplify MITF-M gene.
SEQ ID NO: 11: Designed oligonucleotide for use as a primer to amplify ELK4 gene.
SEQ ID NO: 12: Designed oligonucleotide for use as a primer to amplify ELK4 gene.
SEQ ID NO: 13: Designed oligonucleotide for use as a primer to amplify HLF gene.
SEQ ID NO: 14: Designed oligonucleotide for use as a primer to amplify HLF gene.
SEQ ID NO: 15: Designed oligonucleotide for use as a primer to amplify CLOCK gene.
SEQ ID NO: 16: Designed oligonucleotide for use as a primer to amplify CLOCK gene.
SEQ ID NO: 17: Designed oligonucleotide for use as a primer to amplify ELK4 gene.
SEQ ID NO: 18: Designed oligonucleotide for use as a primer to amplify ELK4 gene.
SEQ ID NO: 19: Designed oligonucleotide for use as a primer to amplify HLF gene.
SEQ ID NO: 20: Designed oligonucleotide for use as a primer to amplify HLF gene.
SEQ ID NO: 21: Partial sequence of MITF-M (SEQ ID NO: 2), which is highly homologous to that (SEQ ID NO: 22) of HLF.
SEQ ID NO: 22: Partial sequence of HLF (SEQ ID NO: 4), which is highly homologous to that (SEQ ID NO: 21) of MITF-M.
SEQ ID NO: 23: Partial sequence of MITF-M (SEQ ID NO: 2), which is highly homologous to that (SEQ ID NO: 24) of ELK4.
SEQ ID NO: 24: Partial sequence of ELK4 (SEQ ID NO: 6), which is highly homologous to that (SEQ ID NO: 23) of MITF-M.
SEQ ID NO: 25: Partial sequence of MITF-M (SEQ ID NO: 2), which is highly homologous to that (SEQ ID NO: 27) of CLOCK.
SEQ ID NO: 26: Partial sequence of MITF-M (SEQ ID NO: 2), which is highly homologous to that (SEQ ID NO: 28) of CLOCK.
SEQ ID NO: 27: Partial sequence of CLOCK (SEQ ID NO: 8), which is highly homologous to that (SEQ ID NO: 25) of MITF-M.
SEQ ID NO: 28: Partial sequence of CLOCK (SEQ ID NO: 8), which is highly homologous to that (SEQ ID NO: 26) of MITF-M.
SEQ ID NO: 29: Partial sequence of MITF-M (SEQ ID NO: 2), which is highly homologous to that (SEQ ID NO: 31) of HLF.
SEQ ID NO: 30: Sequence being homologous to the partial sequence (SEQ ID NO: 29) ofMITF-M and that (SEQ ID NO: 31) ofHLF. (5)..(5): Xaa can be any amino acid residue.
SEQ ID NO: 31: Partial sequence of HLF (SEQ ID NO: 4), which is highly homologous to that (SEQ ID NO: 29) of MITF-M.
SEQ ID NO: 32: Sequence being homologous to the partial sequence (SEQ ID NO: 23) of MITF-M and that (SEQ ID NO: 24) of ELK4. (4)..(4): Xaa can be any amino acid residue or can be being deleted.
SEQ ID NO: 33: Partial sequence of MITF-M (SEQ ID NO: 2), which is highly homologous to that (SEQ ID NO: 35) of CLOCK.
SEQ ID NO: 34: Sequence being homologous to the partial sequence (SEQ ID NO: 33) of MITF-M and that (SEQ ID NO: 35) of CLOCK. (2)..(2): Xaa can be any amino acid residue. (3)..(3): Xaa can be any amino acid residue. (4)..(4): Xaa can be any amino acid residue. (6)..(6): Xaa can be any amino acid residue. (7)..(7): Xaa can be any amino acid residue. (8)..(8): Xaa can be any amino acid residue. (9)..(9): Xaa can be any amino acid residue. (10)..(10): Xaa can be any amino acid residue. (11)..(11): Xaa can be any amino acid residue. (12)..(12): Xaa can be any amino acid residue. (14)..(14): Xaa can be any amino acid residue. (15)..(15): Xaa can be any amino acid residue. (18)..(18): Xaa can be any amino acid residue. (19)..(19): Xaa can be any amino acid residue. (20)..(20): Xaa can be any amino acid residue. (22)..(22) Xaa can be any amino acid residue. (23)..(23): Xaa can be any amino acid residue. (29)..(29): Xaa can be any amino acid residue. (30)..(30): Xaa can be any amino acid residue. (31)..(31): Xaa can be any amino acid residue. (33)..(33): Xaa can be any amino acid residue or can be being deleted. (34)..(34): Xaa can be any amino acid residue. (36)..(36): Xaa can be any amino acid residue. (37)..(37): Xaa can be any amino acid residue. (38)..(38): Xaa can be any amino acid residue. (40)..(40): Xaa can be any amino acid residue or can be being deleted. (41)..(41): Xaa can be any amino acid residue or can be being deleted. (42)..(42): Xaa can be any amino acid residue. (44)..(44): Xaa can be any amino acid residue. (46)..(46): Xaa can be any amino acid residue. (48)..(48): Xaa can be any amino acid residue. (49)..(49): Xaa can be any amino acid residue. (51)..(51): Xaa can be any amino acid residue. (53)..(53): Xaa can be any amino acid residue. (54)..(54): Xaa can be any amino acid residue.
SEQ ID NO: 35: Partial sequence of CLOCK (SEQ ID NO: 8), which is highly homologous to that (SEQ ID NO: 33) of MITF-M.
SEQ ID NO: 36: Partial sequence of MITF-M (SEQ ID NO: 2), which is highly homologous to that (SEQ ID NO: 38) of CLOCK.
SEQ ID NO: 37: Sequence being homologous to the partial sequence (SEQ ID NO: 36) of MITF-M and that (SEQ ID NO: 38) of CLOCK. (2)..(2): Xaa can be any amino acid residue. (3)..(3): Xaa can be any amino acid residue. (5)..(5): Xaa can be any amino acid residue. (8)..(8): Xaa can be any amino acid residue. (9)..(9): Xaa can be any amino acid residue. (13)..(13): Xaa can be any amino acid residue.
SEQ ID NO: 38: Partial sequence of CLOCK (SEQ ID NO: 8), which is highly homologous to that (SEQ ID NO: 36) of MITF-M.

## Claims

1. A method of inhibiting production of BCL2 (B-cell CLL/Lymphoma 2) gene product, comprising inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

2. A method of inhibiting production of BCL2 (B-cell CLL/Lymphoma 2) gene product, comprising using an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

3. A method of inducing cell death of melanoma cells, comprising inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

4. A method of inducing cell death of melanoma cells, comprising using an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

5. A method of identifying a compound that inhibits binding of a protein (protein A) selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M), comprising contacting a compound with protein A and/or MITF-M under conditions that allow for interaction of the compound with protein A and/or MITF-M, employing a system using a signal and/or marker generated by binding of protein A to MITF-M; and detecting presence or absence or change of the signal and/or marker to determine whether the compound inhibits the binding of protein A to MITF-M.

6. A method of identifying a compound that inhibits production of BCL2 (B-cell CLL/Lymphoma 2) gene product, comprising contacting a compound with a protein (protein A) selected from a group consisting of
(i) HLF (hepatic leukemia factor),
(ii) ELK4 (ETS-domain protein Elk-4), and
(iii) CLOCK (circadian locomoter output cycles kaput protein)
and/or MITF-M (microphthalmia-associated transcription factor isoform MITF-M) under conditions that allow for binding of protein A to MITF-M and for interaction of the compound with protein A and/or MITF-M, and determining whether the compound inhibits production of BCL2 (B-cell CLL/Lymphoma 2) gene product.

7. A method of identifying a compound that induces cell death of melanoma cells, comprising contacting a compound with a protein (protein A) selected from a group consisting of
(i) HLF (hepatic leukemia factor),
(ii) ELK4 (ETS-domain protein Elk-4), and
(iii) CLOCK (circadian locomoter output cycles kaput protein)
and/or MITF-M (microphthalmia-associated transcription factor isoform MITF-M) under conditions that allow for binding of protein A to MITF-M and for interaction of the compound with protein A and/or MITF-M, and determining whether the compound inhibits cell death of melanoma cells.

8. An agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

9. An agent for inhibiting production of BCL2 (B-cell CLL/Lymphoma 2) gene product, which inhibits binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

10. An agent for inhibiting production of BCL2 (B-cell CLL/Lymphoma 2) gene product, containing an effective amount of an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalinia-associated transcription factor isoform MITF-M).

11. An agent for inducing cell death of melanoma, which inhibits binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

12. An agent for inducing cell death of melanoma, containing an effective amount of an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

13. An agent for preventing and/or treating a disease accompanied by enhanced production of BCL2 (B-cell CLL/Lymphoma 2) gene product, which inhibits binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

14. An agent for preventing and/or treating a disease accompanied by enhanced production of BCL2 (B-cell CLL/Lymphoma 2) gene product, containing an effective amount of an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

15. An agent for preventing and/or treating a disease accompanied by enhanced production of BCL2 (B-cell CLL/Lymphoma 2) gene product, containing the inhibiting agent according to any one of claims 8 to 10 and/or the agent for inducing cell death according to claim 11 or claim 12.

16. The preventing and/or treating agent according to any one of claims 13 to 15, wherein the disease accompanied by enhanced production of BCL2 (B-cell CLL/Lymphoma 2) gene product is melanoma.

17. A method of preventing and/or treating a disease accompanied by enhanced production of BCL2 (B-cell CLL/Lymphoma 2) gene product, comprising inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

18. A method of preventing and/or treating a disease accompanied by enhanced production of BCL2 (B-cell CLL/Lymphoma 2) gene product, comprising using an agent for inhibiting binding of protein selected from a group consisting of
(i) HLF (hepatic leukemia factor),
(ii) ELK4 (ETS-domain protein Elk-4), and
(iii) CLOCK (circadian locomoter output cycles kaput protein),
to MITF-M (microphthalmia-associated transcription factor isoform MITF-M).

19. A method of preventing and/or treating a disease accompanied by enhanced production of BCL2 (B-cell CLL/Lymphoma 2) gene product, comprising using the inhibiting agent according to any one of claims 8 to 10 and/or the agent for inducing cell death according to claim 11 or claim 12.

20. The preventing and/or treating method according to any one of claims 17 to 19, wherein the disease accompanied by enhanced production of BCL2 (B-cell CLL/Lymphoma 2) gene product is melanoma.

21. A method of treating melanoma, comprising using the treating agent according to claim 16 together with dacarbazine (DTIC).

22. A reagent kit, containing at least one member of a protein (protein A) selected from a group consisting of
(i) HLF (hepatic leukemia factor),
(ii) ELK4 (ETS-domain protein Elk-4), and
(iii) CLOCK (circadian locomoter output cycles kaput protein),
a polynucleotide encoding the protein A, a recombinant vector containing the polynucleotide and a transformant containing the recombinant vector; and at lease one member of MITF-M (microphthalmia-associated transcription factor isoform MTTF-M), a polynucleotide encoding MITF-M, a recombinant vector containing the polynucleotide and a transformant containing the recombinant vector.
